(19) Europäisches Patentamt
European Patent Office
Office européen des brevets

(11) EP 2 084 272 B1

(12) # EUROPÄISCHE PATENTSCHRIFT

(45) Veröffentlichungstag und Bekanntmachung des Hinweises auf die Patenterteilung:
**13.04.2016 Patentblatt 2016/15**

(21) Anmeldenummer: **07818345.6**

(22) Anmeldetag: **21.09.2007**

(51) Int Cl.:
***C12N 9/20*** *(2006.01)*

(86) Internationale Anmeldenummer:
**PCT/EP2007/008256**

(87) Internationale Veröffentlichungsnummer:
**WO 2008/040465 (10.04.2008 Gazette 2008/15)**

(54) **KLONIERUNG, EXPRESSION UND VERWENDUNG SAURER LYSOPHOSPHOLIPASEN**

CLONATION, EXPRESSION AND USE OF ACID LYSOPHOSPHOLIPASES

CLONAGE, EXPRESSION ET UTILISATION DE LYSOPHOSPHOLIPASES ACIDES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **02.10.2006 DE 102006046857**

(43) Veröffentlichungstag der Anmeldung:
**05.08.2009 Patentblatt 2009/32**

(73) Patentinhaber: **AB Enzymes GmbH
64293 Darmstadt (DE)**

(72) Erfinder:
- **NGUYEN, Khanh, Q.
64385 Reichelsheim (DE)**
- **MARSCHNER, Volker
64404 Bickenbach (DE)**
- **TITZE, Kornelia
64367 Mühltal (DE)**
- **WINTER, Bruno
70190 Stuttgart (DE)**

(74) Vertreter: **Hiebl, Inge Elisabeth
Kraus & Weisert
Patentanwälte PartGmbB
Thomas-Wimmer-Ring 15
80539 München (DE)**

(56) Entgegenhaltungen:
**EP-A- 0 808 903**

- **SHEN D-K ET AL: "Characterisation and expression of phospholipases B from the opportunistic fungus Aspergillus fumigatus" FEMS MICROBIOLOGY LETTERS, AMSTERDAM, NL, Bd. 239, Nr. 1, 1. Oktober 2004 (2004-10-01), Seiten 87-93, XP004579957 ISSN: 0378-1097 -& DATABASE UniProt [Online] EBI; 11. Juli 2006 (2006-07-11), "Lysophospholipase 3 precursor (EC <A HREF="http://srs.ebi.ac.uk/srsbin/cgi-bin/wgetz?[enzyme-ECNumber:3.1.1.5]+-e">3.1.1.5</A>) (Phospholipase B 3)." XP002463414 gefunden im EBI accession no. UNIPROT:Q6U819 Database accession no. Q6U819**
- **WRIGHT LESLEY C ET AL: "Cryptococcal phospholipases: a novel lysophospholipase discovered in the pathogenic fungus Cryptococcus gattii." THE BIOCHEMICAL JOURNAL 1 DEC 2004, Bd. 384, Nr. Pt 2, 1. Dezember 2004 (2004-12-01), Seiten 377-384, XP002463390 ISSN: 1470-8728**
- **FUJINO SHUJI ET AL: "Purification and characterization of phospholipase B from Candida utilis" BIOSCIENCE BIOTECHNOLOGY AND BIOCHEMISTRY, Bd. 70, Nr. 2, Februar 2006 (2006-02), Seiten 377-386, XP002463398 ISSN: 0916-8451**
- **GHANNOUM MAHMOUD A: "Potential role of phospholipases in virulence and fungal pathogenesis" CLINICAL MICROBIOLOGY REVIEWS, Bd. 13, Nr. 1, Januar 2000 (2000-01), Seiten 122-143, XP002463391 ISSN: 0893-8512**

EP 2 084 272 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- LEIDICH STEVEN D ET AL: "Cloning and disruption of caPLB1, a phospholipase B gene involved in the pathogenicity of Candida albicans" JOURNAL OF BIOLOGICAL CHEMISTRY, Bd. 273, Nr. 40, 2. Oktober 1998 (1998-10-02), Seiten 26078-26086, XP002463392 ISSN: 0021-9258
- HOOVER CHARLES I ET AL: "Cloning and regulated expression of the Candida albicans phospholipase B (PLB1) gene" FEMS MICROBIOLOGY LETTERS, Bd. 167, Nr. 2, 15. Oktober 1998 (1998-10-15), Seiten 163-169, XP002463393 ISSN: 0378-1097
- HAKI G D ET AL: "Developments in industrially important thermostable enzymes: A review." BIORESOURCE TECHNOLOGY, Bd. 89, Nr. 1, August 2003 (2003-08), Seiten 17-34, XP002463394 ISSN: 0960-8524
- KOHLER ET AL: "Phospholipase A2 and Phospholipase B activities in fungi" BIOCHIMICA AND BIOPHYSICA ACTA. MOLECULAR AND CELL BIOLOGY OF LIPIDS, ELSEVIER, AMSTERDAM, NL, Bd. 1761, Nr. 11, 15. November 2006 (2006-11-15), Seiten 1391-1399, XP005767812 ISSN: 1388-1981

## Beschreibung

Die Erfindung betrifft neue DNA-Sequenzen, die Polypeptide mit Lysophospholipaseaktivität codieren. Die Erfindung betrifft ferner neue Polypeptide mit Lysophospholipaseaktivität. Bei diesen Polypeptiden handelt es sich um saure Lysophospholipasen mit hoher Thermostabilität. Ferner betrifft die Erfindung auch die Verwendung dieser Lysophospholipasen zur Verbesserung der Filtrierbarkeit von Sirupen aus Weizenstärke, Verbesserung von Teigen, etc.

Phospholipide, wie Lecithin oder Phosphatidylcholin, bestehen aus Glycerol verestert mit zwei Fettsäuren an der äußeren (sn-1) und an der mittleren (sn-2) Position des Glycerols, sowie einer estergebundenen Phosphatgruppe an der dritten Position (sn-3). Die Phosphatgruppe ihrerseits kann wiederum z.B. mit Aminoalkoholen verestert sein. Phospholipasen katalysieren die Hydrolyse der Acylbindung oder der Esterbindungen der Phospholipide. Bei den Phospholipasen gibt es verschiedene Typen, die sich in ihrem Spaltmuster unterscheiden. Bei den acylspaltenden Phospholipasen unterscheidet man die Phospholipasen A1 und A2 die entweder die Acylgruppe an der sn-1 oder der sn-2 Position hydrolysieren und dabei Lysophospholipid produzieren. Aus diesem Grund kann durch Lysophospholipase (LPL) die verbleibende Fettsäure hydrolysiert werden. Für die Lysophospholipasen ist keine Positionsselektivität bekannt.

In der Literatur sind Phospholipasen Typ B beschrieben die z.T. quasi simultan beide Acylgruppen hydrolysieren, ohne dass die Bildung eines Intermediates an Lysolecithin beobachtet werden kann (FEMS Microbiol. Let. 18 (1983) 15-18; Annu. Rev. Biochem. 41 (1972) 129-160). Dies ist oft wie z.B. bei der PLB1 und PLB3 Aktivität von *Saccharomyces cerevisiae* (Biochemistry 1999 May 4; 38(18):5864-5871) dadurch bedingt, dass das Enzym viel mehr LPL-Aktivität als $PLA_n$-Aktivität hat. Reine Lysophospholipasen ohne Phospholipasenebenaktivität können keine Fettsäuren aus Phospholipiden abspalten, die Fettsäuren an den Positionen sn-1 und sn-2 enthalten.

Die vorstehend beschriebenen Lysophospholipasen des Typs B, von denen die Aminosäure- und/oder Nucleinsäuresequenz bekannt ist, lassen sich in 2 Gruppen einteilen. Bei ihnen handelt es sich um Lysophospholipasen die zusätzlich eine sehr geringe Phospholipase A-Aktivität aufweisen können.

a) Es sind die Sequenzen von mehreren Enzymen mit Molekulargewichten von 45-100 kDa bekannt. Dazu gehören die PLB aus *Aspergillus niger* (WO 01/27251, WO 03/097825), die PLB aus *Aspergillus fumigatus* (Shen et al. FEMS Microbiol Lett. 2004, 239 (1):87-93), die PLB aus *Aspergillus oryzae* (WO 01/27251 & WO 01/29222), die PLB aus *Fusarium venenatum* und *Fusarium verticillioides* (WO 00/28044), die PLB aus *Penicillium notatum* auch *P. chrysogenum* genannt (N. Masuda et al., Eur. J. Biochem., 202: 783-787 (1991)), die PLB 1-3 aus *Saccharomyces cerevisiae* (Lee et al., 1994 J. Biol. Chem. 269: 19725-19730, Merkel et al., 1999 J. Biol. Chem. 274: 28121-28127), die PLB aus *Torulaspora delbrueckii* (alter Name *Saccharomyces rosei*) (Watanabe et al., 1994, FEMS Microbiology Letters 124: 29-34), *Kluyveromyces lactis* (Oishi et al., 1999 Biosci. Biotechnol. Biochem. 63: 83-90), *Neurospora crassa* (EMBL 042791) und *Schizosaccharomyces pombe* (EMBL O13857).

b) Es sind mehrere Sequenzen von Enzymen mit einem Molekulargewicht von 30-40 kDa bekannt. Dazu gehören die Lysophospholipase aus *Aspergillus foetidus,* EP 0 808 903, die PLB aus *A. niger,* WO 01/27251 und WO 03/097825, die PLB1 und PLB2 aus *Candida albicans* (J. Biol. Chem. 273 (40): 26078-26086, 1998, Medical Mycology 37:61-67, 1998), und die PLB aus *Pseudomonas* PS21, JP 03151879.
Des Weiteren sind in der Literatur Phospholipasen Typ A erwähnt.

c) Die Gruppe der Phospholipasen Typ A mit einem Molekulargewicht von ca. 30-40 kDa. Dazu gehören folgende Phospholipasen aus dem Stand der Technik: In der WO 98/31790 (AB Enzymes GmbH) wird beschrieben, dass in *Aspergillus niger* eine geeignete Phospholipase A zur Entschleimung von Speiseöl gefunden wurde. Das Protein (36 kDa) entfaltet Phospholipaseaktivität nur nach proteolytischer Spaltung, wobei die zwei Fragmente (30 + 6 kDa) über Disulfidbrücken verbunden bleiben. Das Enzym spaltet Lecithin zu Lysolecithin, ist aber auch in der Lage Lysolecithin weiter zu spalten z.B. zum Phosphatidylcholin. In der WO 98/26057 wird eine Phospholipase A aus *Fusarium* sp. beschrieben mit einem Molekulargewicht von 29 $\pm$ 10 kDa und einem isoelektrischen Punkt zwischen pl 4.5-8. In der JP 10-155493 A2 wird eine Phospholipase A1 aus A. *oryzae* (295 aa) beschrieben; aus der WO 02/24881 ist eine Phospholipase A aus der Hefe *Zygosascus hellenicus* (407 aa) mit einem isoelektrischen Punkt pl von ca. 4,2 bekannt und in der JP 03151879 wird eine bakterielle Phospholipase aus *Pseudomonas* sp. mit einem Molekulargewicht von ca. 30 kDa beschrieben.
Weiterhin werden in der EP 0 575 133 A2 (US 5,538,874, US 5,378,623, US 5,521,080) Phospholipasen A1 aus *Aspergillen* mit einem Molekulargewicht von 30-40 kDa und einem pl von 2,8-4,5 beschrieben, jedoch ohne Angabe von Sequenzinformationen. Ferner enthalten diese Patentschriften keine Hinweise oder Strategien zum Erhalt der zugehörigen DNA durch Klonierung.

d) Die Gruppe der Phospholipasen Typ A mit einem Molekulargewicht von ca. 60-100 kDa. Dazu gehören: Phospholipasen aus *Hyphozyma,* einer hefeähnlichen Pilzart, beschrieben in WO 98/18912 und Phospholipasen aus *Aspergillus niger,* gemäß WO 03/097825.

Darüber hinaus beschreibt die WO 2004/097012 "Kernpeptide" bekannter Phospholipasen A2 mit einer erhöhten Phospholipaseaktivität.

WO 00/32758, WO 03/060112 und WO 2004/111216 beschreiben Methoden mittels "Protein Engineering" bekannter Lipasen, z.B. aus *Thermomyces lanuginosus* und anderer Phospholipasen zu Enzymvarianten zu gelangen, die eine veränderte Lipase- bzw. Phospholipaseaktivität aufweisen.

Die WO 02/066622 beschreibt neue Gene mit hoher Homologie zu den Genen der *Thermomyces lanuginosus* Lipase sowie deren Verwendung für das gene shuffling zur Erzeugung neuer lipolytischer Enzyme.

WRIGHT LESLEY C ET AL: "Cryptococcal phospholipases: a novel lysophospholipase discovered in the pathogenic fungus Cryptococcus gattii.", BIOCHEMICAL JOURNAL 1 DEC 2004, Bd. 384, Nr. Pt 2, 1. Dezember 2004, Seiten 377-384, XP002463390 beschreibt die Reinigung einer Lysophospholipase aus Cryptococcus gattii, die ein 66 kDa Glycoprotein ist und bei hohen Temperaturen tief ist.

EP 0 808 903 beschreibt eine rekombinant hergestellte Lysophospholipase aus Aspergillus, bevorzugt Aspergillus foetidus, sowie deren Verwendung zur Verbesserung der Filtrationsleistung von Stärkehydrolysaten.

FUJINO SHUJI ET AL: "Purification and characterization of phospholipase B from Candida utilis", BIOSCIENCE BIOTECHNOLOGY AND BIOCHEMISTRY, Bd. 70, Nr. 2, Februar 2006 (2006-02), Seiten 377-386, XP002463398 beschreibt die Reinigung und Charakterisierung einer Phospholipase B auf Candida utilis. GHANNOUM MAHMOUD A: "Potential role of phospholipases in virulence and fungal pathogenesis", CLINICAL MICROBIOLOGY REVIEWS, Bd. 13, Nr. 1, Januar 2000, Seiten 122-143, XP002463391 beschreibt Anwendungen von Pilzphospholipasen, insbesondere im Hinblick auf deren Virulenz.

LEIDICH STEVEN D ET AL: "Cloning and disruption of caPLB1, a phospholipase B gene involved in the pathogenicity of Candida albicans", JOURNAL OF BIOLO-GICAL CHEMISTRY, Bd. 273, Nr. 40, 2. Oktober 1998, Seiten 26078-26086, XP002463392 beschreibt die Klonierung und Expression einer Candida albicans Phospholipase B.

HOOVER CHARLES I ET AL: "Cloning and regulated expression of the Candida albicans phospholipase B (PLB1) gene", FEMS MICROBIOLOGY LETTERS, Bd. 167, Nr. 2, 15. Oktober 1998, Seiten 163-169, XP002463393 beschreibt Enzymsubstratwechselwirkungen zwischen Candida-Phospholipasen und Wirtszellphospholipasen.

Lysophospholipasen werden im Prozess der Glucosesirupherstellung und weiterer daraus abgeleiteter Produkte eingesetzt um die Abtrennung von unerwünschten Bestandteilen zu erleichtern (siehe EP 0 219 269 B1). Diese Bestandteile werden durch ein Filtrationsverfahren abgetrennt, bei dem häufig nur trübe Filtrate erhalten werden. Durch den Einsatz von Lysophospholipasen kann sowohl die Filtrationsgeschwindigkeit, wie auch die Klarheit des Filtrates positiv beeinflusst werden. Auf Grund der hohen Viskosität der Sirupe (Stärkepartialhydrolysat, bevorzugt Weizenstärkepartialhydrolysat) müssen diese bei hohen Temperaturen gehalten und weiter verarbeitet werden. Dies erfordert Enzyme mit einer hohen Temperaturstabilität.

Der vorliegenden Erfindung liegt somit die Aufgabe zugrunde, Proteine bzw. Polypeptide mit verbesserten Lysophospholipaseeigenschaften bereitzustellen. Die neuen Lysophospholipasen sollen insbesondere ein hohes Verhältnis von Lysophospholipase zu Phospholipaseaktivität aufweisen. Ebenso sollen die Proteine mit Lysophospholipaseaktivität eine erhöhte Thermostabilität besitzen.

Ferner sollen die Proteine mit Lysophospholipaseaktivität einfach, kostengünstig und wirtschaftlich zu produzieren sein. Ferner sollen erfindungsgemäß Expressionskonstrukte bereitgestellt werden, die sich zur Produktion der Proteine mit Lysophospholipaseaktivität eignen.

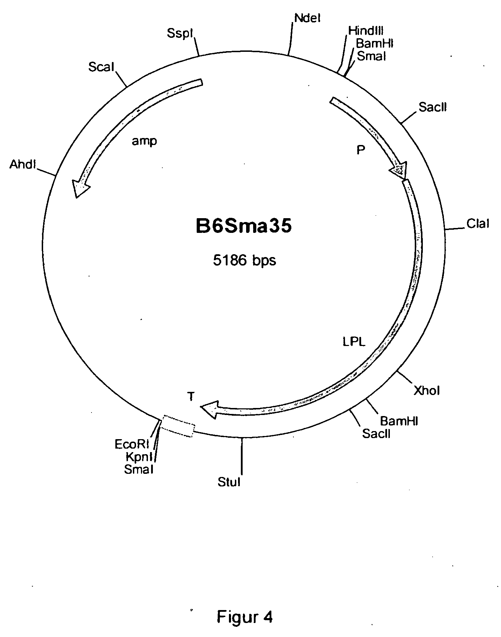

Die vorstehenden Aufgaben werden gelöst durch eine DNA-Sequenz, die ein Polypeptid mit Lysophospholipaseaktivität codiert, dadurch gekennzeichnet, dass die DNA-Sequenz ausgewählt ist aus a) DNA-Sequenzen, die eine Nucleotidsequenz gemäß SEQ ID NO: 1 umfassen, b) DNA-Sequenzen, die die codierende Sequenz gemäß SEQ ID NO: 1 umfassen, c) DNA-Sequenzen, die die Proteinsequenz gemäß SEQ ID NO: 2 codieren, d) DNA-Sequenzen, die von dem Plasmid B6Sma35 mit der Restriktionskarte gemäß Figur 4 und hinterlegt unter der Hinterlegungsnummer DSM 18370 codiert werden, e) DNA-Sequenzen, die aufgrund der Degeneriertheit des genetischen Codes mit den DNA-Sequenzen gemäß a), b), c) oder d) verwandt sind, und f) komplementären Strängen zu den Sequenzen gemäß a) bis e), wobei die DNA-Sequenz bevorzugt aus *Aspergillus* und noch bevorzugter aus *Aspergillus fumigatus* abgeleitet ist und wobei das Polypeptid mit Lysophospholipaseaktivität bei einer Temperatur von 65°C, pH 5 in Weizenstärkepartialhydrolysat, über mindestens 4 h eine Aktivität von mindestens 80% beibehält.

Die Erfindung betrifft ferner ein Polypeptid mit Lysophospholipaseaktivität, ausgewählt aus

a) einem Polypeptid, das von dem codierenden Teil einer DNA-Sequenz nach einem der Ansprüche 1 bis 2 codiert wird,

b) einem Polypeptid mit einer Sequenz, die mindestens 89% Identität zu den Aminosäuren 16 bis 611 von SEQ ID NO: 2 aufweist, wobei das Polypeptid mit Lysophospholipaseaktivität bei einer Temperatur von 65°C, pH 5 in Weizenstärkepartialhydrolysat, über mindestens 4 h eine Aktivität von mindestens 80% beibehält.

Weiterhin betrifft die Erfindung Expressionskonstrukte bzw. Wirte, die in der Lage sind, die erfindungsgemäßen Polypeptide mit Lysophospholipaseaktivität zu exprimieren. Ferner betrifft die Erfindung auch die entsprechenden Expressionsplasmide und Vektoren. Weiterhin betrifft die Erfindung die Verwendung der erfindungsgemäßen Polypeptide für Anwendungen im Bereich der Lebensmitteltechnologie, insbesondere zur Bearbeitung von Stärkehydrolyse-Produkten aus Weizenstärke.

Die im vorstehenden Stand der Technik genannten Sequenzen von Lysophospholipasen sind vom Schutzumfang der Erfindung expressis verbis ausgenommen. Insbesondere sind vom Schutzumfang der Erfindung die Sequenzen der Proteine mit Lysophospholipaseaktivität sowie die entsprechenden DNA-Sequenzen der folgenden Dokumente ausgenommen: WO 01/27251, WO 2004/111216, WO 2004/097012, WO 03/060112, WO 02/24881, WO 00/28044, WO 00/32758, WO 03/097825, EP 999 73 065.8 sowie dazugehörige Teilanmeldungen. Die Ausnahme der Sequenzen betrifft diese Druckschriften in ihrer Gesamtheit sowie einzeln und in beliebigen Kombinationen.

Überraschenderweise wurde gefunden, dass eine DNA-Sequenz, die ein Polypeptid mit Lysophospholipaseaktivität codiert, das ein hohes Molekulargewicht, ein hohes Lysophospholipase zu Phospholipaseaktivitätsverhältnis sowie eine erhöhte Thermostabilität besitzt, aus einem Stamm der Gattung *Aspergillus fumigatus* isoliert werden kann. Bei dieser Lysophospholipase handelt es sich um eine saure, von einem Pilz abstammende Lysophospholipase mit einem Molekulargewicht von circa 64 bis 78 kDa, die Fettsäure aus Lysolecithin hydrolysieren kann, ein hohes Verhältnis Lysophospholipaseaktivität zu Phospholipaseaktivität und eine erhöhte Thermostabilität besitzt und aus einem Organismus der Gattung *Aspergillus* isolierbar ist.

Dabei ist unter erhöhter Thermostabilität zu verstehen, dass das Enzym bei einer Temperatur von 65°C, pH 5 in Weizenstärkepartialhydrolysat, über mindestens 4 h eine Aktivität von mindestens 80% beibehält.

Die erhöhte Thermostabilität der erfindungsgemäßen Lysophospholipase war überraschend, ebenso das hohe Lysophospholipase zu Phospholipaseaktivitätsverhältnis und aufgrund der im Stand der Technik beschriebenen Lysophospholipasen nicht naheliegend. Zu keiner der im Stand der Technik beschriebenen natürlich vorkommenden Lysophospholipasen sind diese Eigenschaften beschrieben oder auch nur nahegelegt.

Da es keine Veröffentlichung zu thermostabilen Lysophospholipasen aus filamentösen Pilzen gibt und damit auch keine Anhaltspunkte welche Strukturelemente (Helices, β-Faltblätter, Loops) bei einer Lysophospholipase speziell gestaltet sein müssen (vorliegen von ionischen Wechselwirkungen über geladene Aminosäuren, Disulfidbrücken, Van-der-Waals Wechselwirkungen, hydrophobe Wecheselwirkungen), um eine hohe Thermostabilität zu gewährleisten, war es nicht vorherzusehen, dass die gefundene Lysophospholipase diese Eigenschaften besitzen würde. Ebenso ist es nicht hinreichend bekannt, wo und wie Änderungen an einem lipolytischen Polypeptid durchgeführt werden müssen um ein hohes Lysophospholipase zu Phospholipaseaktivitätsverhältnis zu erhalten. Die Gene der Familie der Lipasen im weitesten Sinne, zu denen auch die Phospholipasen und Lysophospholipasen gehören, zeigen, dass kleine Veränderungen an der Sequenz die Eigenschaften des von dieser Sequenz codierten Enzyms stark beeinflussen. Dies ist z.B. in der Anmeldung WO 03/060112 gezeigt. Diese Anmeldung beschreibt ein Verfahren zur Erzeugung von Varianten von lipolytischen Enzymen. Dabei werden die Änderungen in der Substratspezifität über random-Mutagenese erreicht, nicht über eine spezifische, gerichtete, Mutationen. Diese Anmeldung zeigt auch, dass trotz hoher Homologie in der Sequenz die Eigenschaft des davon codierten Enzyms nicht vorhergesagt werden kann. Diese mangelnde Korrelation zwischen DNA-Sequenz und codiertem Enzym sowie Unterschiede in der Codonusage durch die einzelnen Stämme erlauben keine Ableitung von Primern aus bekannten Sequenzen zum Auffinden von neuen Sequenzen mit gezielten Eigenschaften, wie hohe Thermostabilität, geringe Lipaseaktivität, hohes Phospholipase zu Lysophospholipaseaktivitätsverhältnis und umgekehrt. Weiterhin ist es durch die hohe Homologie zwischen Phospholipasen und Lysophospholipasen nicht vorhersehbar gewesen, dass mit dem gewählten Ansatz eine Lysophospholipase mit den genannten Eigenschaften isoliert wird. Die für Primeransätze möglichen Regionen zeigen auf der Aminosäureebene kurze Sequenzteile von 2 bis 3 konservierten Aminosäuren mit Unterbrechungen durch variable Aminosäuren. Bei den variablen Aminosäuren gibt es keine Tendenz, welche Aminosäuren bevorzugt in Phospholipasen und welche bevorzugt in Lysophopsholipasen zu finden sind. Zusammen mit der stammspezifischen Codonusage zeigen sich daher auf DNA-Ebene keine Konsensusregionen die für eine gezielte Isolierung von Lysophospholipasen genutzt werden können. Die für die Primer gewählten DNA-Regionen sind daher auch nicht vorhersagbar der Teil des Enzyms, in dem die spezifischen, für die genannten Eigenschaften codierenden Aminosäuren lokalisiert sind. Die gewählten Primeransätze waren nicht deckungsgleich mit bekannten Lysophospholipasen. Daher ist es umso überraschender, dass das gefundene Polypeptid ein hohes Lysophospholipase zu Phospholipaseaktivitätsverhältnis aufweist.

Die erfindungsgemäße Lysophospholipasesequenz gemäß SEQ ID NO: 1 wurde mit Lysophospholipasesequenzen aus dem Stand der Technik verglichen. Die Sequenz SEQ ID NO: 1 zeigt die höchste Identität von 88% mit der Sequenz PLB3 aus *Aspergillus fumigatus* CBS 14489 (Shen et al., FEMS Microbiol. Letters 2004, 239 (1): 87-93). Eine hohe Übereinstimmung auf der Ebene der Aminosäuresequenz wurde auch zu Sequenz 13 aus WO 03/097825 bekannten Aminosäuresequenz aus *Aspergillus niger* (68%), sowie der LPL1 aus *Aspergillus oryzae,* WO 01/027251 (73%) gefunden. Das erfindungsgemäße Enzym zeichnet sich weiterhin im Gegensatz zu den im Stand der Technik beschriebenen Enzymen durch ein hohes Verhältnis Lysophospholipase zu Phospholipaseaktivität aus.

Die Erfindung betrifft somit auch Polypeptide mit Lysophospholipaseaktivität mit einer Sequenz, die mindestens 89% Identität zu der Sequenz gemäß SEQ ID NO: 1 besitzt. Bevorzugt betrifft die Erfindung ein Polypeptid mit Lysophospholipaseaktivität mit einer Sequenz, die mindestens 89% Identität zu den Aminosäuren 16 bis 611 von SEQ ID NO: 2 besitzt. Bevorzugt beträgt der Identitätsgrad mindestens 90%, bevorzugter mindestens 93%, noch bevorzugter mindestens 95% und besonders bevorzugt mindestens 98%, unter der Bedingung, dass die jeweiligen Sequenzen Lysophospholipaseaktivität aufweisen.

Der Grad der Sequenzidentität wird dabei bevorzugt so bestimmt, dass man die Anzahl der Reste der kürzeren Sequenz, die an dem Vergleich beteiligt ist und die ein "entsprechendes" Gegenstück in der anderen Sequenz besitzt, ermittelt. Für die Zwecke der vorliegenden Erfindung wird die Identität dabei bevorzugt in üblicher Weise unter Verwendung der üblichen Algorithmen bestimmt. Erfindungsgemäß werden zum Vergleich nur die cDNAs bzw. Aminosäuren der jeweiligen reifen Proteine herangezogen. Als homologe Sequenzen wurden erfindungsgemäß ähnliche, bevorzugt identische, Sequenzgegenstücke mit Hilfe von bekannten Computerprogrammen ermittelt. Ein Beispiel für ein derartiges Programm ist das Programm Clone Manager Suite, das den Programmteil Align Plus enthält und von Scientific & Educational Software, Durham, NC, USA vertrieben wird. Dabei wird ein Vergleich zweier wie vorstehend definierter DNA- bzw. Aminosäuresequenzen durchgeführt, unter der Option local alignment entweder nach der Methode FastScan - MaxScore oder nach der Methode Needleman-Wunsch unter Beibehaltung der Defaultwerte. Speziell wurde erfindungsgemäß zur Berechnung der Identität die Programmversion "Clone Manager 7 Align Plus 5" mit den Funktionen "Compare Two Sequences/Local Fast Scan-Max Score/Compare DNA sequences", bzw. für Aminosäuren "Compare Two Sequences/Global/Compare sequences as Amino Acids" angewendet. Dabei wurden die aus den folgenden Quellen zugänglichen Algorithmen verwendet: Hirschberg, D.S. 1975. A linear space algorithm for computing longest common subsequences. Commun Assoc Comput Mach 18:341-343; Myers, E.W. and W. Miller. 1988. Optimal alignments in linear space. CABIOS 4:1, 11-17; Chao, K-M, W.R. Pearson and W. Miller. 1992. Aligning two sequences within a specified diagonal band. CABIOS 8:5, 481-487.

Die Erfindung betrifft ferner Additions- und/oder Deletionsmoleküle der vorstehenden Polypeptide mit Lysophospholipaseaktivität. So kann ein erfindungsgemäß modifiziertes Polypeptid mit Lysophospholipaseaktivität durch Anfügen weiterer Sequenzen am N-terminalen und/oder C-terminalen Ende verlängert werden, wobei die so erhaltenen Aminosäuresequenzen noch Lysophospholipaseaktivität aufweisen müssen. Dadurch können Hybridmoleküle hergestellt werden, die weitere vorteilhafte Eigenschaften besitzen. Beispielsweise können Suspensionsproteine bzw. deren native Vorläuferformen in hohem Maße sekretierte Proteine angefügt werden, wodurch die Sekretionseffizienz weiter verbessert wird. Ferner können aktive Sequenzabschnitte anderer Enzyme hinzugefügt werden, um Enzyme mit mehrfacher Spezifität herzustellen. Ferner können polare oder unpolare Sequenzen angefügt werden, um die Löslichkeitseigenschaften bzw. die Membrangängigkeit des so erhaltenen Enzyms in gezielter Weise zu beeinflussen.

Erfindungsgemäß können auch Sequenzabschnitte des Polypeptids mit Lysophospholipaseaktivität deletiert werden unter Beibehaltung der Lysophospholipaseaktivität. Die Mutationen, Elongationen und Verkürzungen können in an sich bekannter Weise nach auf dem Fachgebiet gut bekannten Verfahren durchgeführt werden. Verkürzte Polypeptide zeichnen sich häufig durch eine verbesserte Sekretionshöhe im Vergleich zu den Volllängenpolypeptiden aus. Sie können auch höhere Thermostabilitäten im Vergleich zum Volllängenpolypeptid aufweisen, da sie nur den "komprimierten Kern" enthalten.

Die Erzeugung solcher Varianten ist allgemein auf dem Fachgebiet bekannt. Beispielsweise können Aminosäuresequenz-Varianten der Polypeptide durch Mutation in der DNA hergestellt werden. Verfahren zur Mutagenese und Nucleotidsequenz-Veränderung sind auf dem Fachgebiet gut bekannt (vgl. beispielsweise Kunkel, Proc. Natl. Acad. Sci. USA, 82:488 (1985), Kunkel et al., Methods in Enzymol., 154:367 (1987), US-Patent Nr. 4,873,192, Walker und Gaastra, eds., Techniques in Molecular Biology, Mac Millan Publishing Company, New York (1983)). Hinweise über geeignete Aminosäuresubstitutionen, die die biologische Aktivität des Proteins von Interesse nicht beeinträchtigen, finden sich in dem Model von Dayhoff et al., Atlas of Protein Sequence and Structure, Natl. Biomed. Res. Found., Washington, D.C. (1978). Konservative Substitutionen wie der Austausch einer Aminosäure gegen eine andere mit ähnlichen Eigenschaften sind bevorzugt. Diese Austausche lassen sich in 2 Hauptgruppen mit zusammen 4 Untergruppen einteilen und ein Austausch in jeder Untergruppe wird als konservativer Austausch bezeichnet, der bevorzugt nicht die Aktivität oder die Faltung des Proteins beeinflusst.

| | | |
|---|---|---|
| Aliphatisch | Nicht-Polar | G A P |
| | | I L V |
| | Polar und ungeladen | C S T M N Q |
| | Polar und geladen | D E |
| | | K R |

(fortgesetzt)

| | | |
|---|---|---|
| Aromatisch | | H F W Y |

Die Ausdrücke "Protein", "Peptid" und "Polypeptid" werden im Wesentlichen austauschbar verwendet. Ein Polypeptid oder Enzym mit Phospholipaseaktivität oder eine Phospholipase soll ein Enzym bezeichnen, dass die Freisetzung von Fettsäuren aus Phospholipiden, zum Beispiel Lecithinen, katalysiert. Die Phospholipaseaktivität kann unter Verwendung an sich bekannter, beliebiger Assays, bei denen eines dieser Substrate verwendet wird, bestimmt werden.

Ein Polypeptid oder Enzym mit Lysophospholipaseaktivität oder eine Lysophospholipase soll ein Enzym bezeichnen, dass die Freisetzung von Fettsäure aus Lysophospholipiden, zum Beispiel Lysolecithinen, katalysiert. Die Lysophospholipaseaktivität kann unter Verwendung an sich bekannter, beliebiger Assays, bei denen eines dieser Substrate verwendet wird, bestimmt werden.

Im Zusammenhang mit den erfindungsgemäßen Polypeptiden soll der Ausdruck "Phospholipase" bzw. Phospholipase A sowohl Enzyme mit Phospholipase A1- als auch Phospholipase A2-Aktivität bezeichnen. Dabei sind Phospholipase A1 bzw. A2 definiert gemäß der Standardenzym-EC-Klassifikation als EC 3.1.1.2 bzw. 3.1.1.4.

Phospholipase B oder Lysophospholipase sind Polypeptide gemäß der Standardenzym-EC-Klassifikation EC 3.1.1.5.

Beschrieben sind ferner DNA-Sequenzen, die ein Polypeptid mit Lysophospholipaseaktivität codieren, die Mutationen, Modifikationen bzw. Variationen der Sequenz gemäß SEQ ID NO: 1 umfassen. Beschrieben sind auch Sequenzen, die unter relaxierten oder stringenten Bedingungen mit den vorstehenden Sequenzen hybridisieren. Als stringente Bedingungen gelten: Hybridisierung bei 65°C, 18 h in Dextransulfat-Lösung (GenescreenPlus, DuPont), danach waschen der Filter jeweils 30 min zuerst mit 6 x SSC, zweimal 2 x SSC, zweimal 2 x SSC, 0,1% SDS und anschließend mit 0, 2 x SSC bei 65°C (Membrane transfer and detection methods, Amersham).

Ferner betrifft die Erfindung auch DNA-Sequenzen, die aufgrund der Degeneriertheit des genetischen Codes mit den vorstehenden erfindungsgemäßen Sequenzen verwandt sind, sowie allylische Varianten davon. Die Degeneriertheit des genetischen Codes kann sich dabei aufgrund natürlicher Degeneriertheit oder aufgrund eines speziell gewählten Codongebrauchs ergeben. Natürlich vorkommende allelische Varianten können unter Verwendung gut bekannter Techniken der Molekularbiologie, wie zum Beispiel der Polymerasekettenreaktion (PCR) und Hybridisierungstechniken, identifiziert werden.

Die Erfindung betrifft ferner ein Verfahren zur Herstellung eines Polypeptids mit Lysophospholipaseaktivität nach rekombinanten Techniken umfassend die Züchtung rekombinanter prokaryotischer und/oder eukaryotischer Wirtszellen, die eine erfindungsgemäße DNA-Sequenz enthalten unter Bedingungen, die die Expression des Enzyms fördern, sowie die anschließende Gewinnung des Enzyms. Die Erfindung betrifft ferner die Verwendung der erfindungsgemäßen Polynucleotidsequenzen zur Herstellung von Sonden zum Auffinden ähnlicher Sequenzen, die entsprechende Enzyme codieren, in anderen Organismen sowie zur Transformation von Wirtszellen.

Eine DNA-Sequenz, die ein erfindungsgemäßes Polypeptid codiert, kann verwendet werden, um beliebige Wirtszellen, wie beispielsweise Zellen von Pilzen, Hefen, Bakterien, Pflanzen oder Säugern zu transformieren. Derart transformierte Zellen zeichnen sich durch eine Sekretion der erfindungsgemäßen Lysophospholipase aus. Das so produzierte Lysophospholipaseenzym bewirkt eine effiziente Hydrolyse der Fettsäuren aus Lysophospholipiden.

Die Erfindung betrifft auch Expressionskassetten, die zur Einschleusung der eine erfindungsgemäße Lysophospholipase codierenden DNA-Sequenz bzw. eines offenen Leserasters in eine Wirtszelle verwendet werden können. Sie umfassen bevorzugt eine Transkriptionsstartregion, die mit dem offenen Leseraster verknüpft ist. Eine derartige Expressionskassette kann eine Vielzahl von Restriktionsspaltstellen zur Insertion des offenen Leserasters und/oder anderer DNAs, z.B. einer Transkriptions-Regulator-Region und/oder selektierbare Markergene enthalten. Die Transkriptionskassette umfasst in der 5'→3'-Richtung der Transkription eine Transkriptions- und Translationsstartregion, die DNA-Sequenz von Interesse und eine Transkriptions- und Translationsstopregion, die in einer mikrobiellen Zelle funktionell ist. Die Terminationsregion kann bezüglich der Transkriptioninitiationsregion nativ sein, kann bezüglich der DNA-Sequenz von Interesse nativ sein oder kann von einer beliebigen anderen Quelle abgeleitet sein.

Der Ausdruck "offenes Leseraster" (ORF) bezeichnet die Aminosäure-Sequenz, die zwischen den Translationsstart- und -Stop-Codons einer codierenden Sequenz codiert wird. Die Ausdrücke "Start-Codon" und "Stop-Codon" bezeichnen eine Einheit aus drei benachbarten Nucleotiden (Codons) in einer codierenden Sequenz, die den Kettenstart und -stop der Proteinsynthese (mRNA-Translation) spezifizieren.

"Funktionelle Verknüpfung" bezeichnet im Zusammenhang mit einer Nucleinsäure eine Verbindung als Teil des gleichen Nucleinsäuremoleküls in geeigneter Positionierung und Orientierung zum Transkriptionsstart des Promotors. DNA in funktioneller Verknüpfung an einen Promotor befindet sich unter der Transkriptions-Initiations-Regulation des Promotors. Codierende Sequenzen können funktionell mit der Regulatorsequenz in Sense- oder Antisense-Orientierung verknüpft sein. Bei Bezugnahme auf Polypeptide bedeutet funktionelle Verknüpfung die Verbindung als Teil desselben Polypeptids, d.h. über Peptidylbindungen.

Erfindungsgemäß können beliebige Promotoren verwendet werden. Promotor bezeichnet die Nucleotidsequenz üblicherweise stromaufwärts (5') bezüglich der codierenden Sequenz und kontrolliert die Expression der codierenden Sequenz, indem die Erkennung für die RNA-Polymerase und anderer Faktoren, die für die richtige Transkription erforderlich sind, bereitgestellt wird. Der erfindungsgemäß verwendete Promotor kann einen Minimalpromotor umfassen, d.h. eine kurze DNA-Sequenz aus einer TATA-Box und anderen Sequenzen, die die Transkriptionsstartstelle spezifizieren, an die Regulatorelemente zur Kontrolle der Expression angefügt sind.

Der erfindungsgemäß verwendete Promotor kann auch eine Nucleotidsequenz umfassen, die einen Minimalpromotor und Regulatorelemente umfasst, der die Expression einer codierenden Sequenz oder funktionellen RNA kontrollieren kann. Dieser Typ von Promotorsequenz besteht aus proximalen und distalen stromaufwärts gelegenen Elementen, wobei die zuletzt genannten Elemente oft als Enhancer bezeichnet werden. Folglich ist ein Enhancer eine DNA-Sequenz, die die Promotor-Aktivität stimulieren kann und kann ein dem Promotor innewohnendes Element oder ein insertiertes heterologes Element sein, um die Expressionshöhe oder Gewebsspezifität eines Promotors zu verstärken. Er kann in beiden Orientierungen funktionieren und kann selbst bei stromaufwärtiger oder stromabwärtiger Platzierung von dem Promotor funktionieren. Sowohl Enhancer als auch andere stromaufwärts gelegene Promotor-Elemente binden sequenzspezifisch DNAbindende Proteine, die ihre Wirkungen vermitteln. Promotoren können in ihrer Gesamtheit von einem nativen Gen abgeleitet sein oder können aus verschiedenen Elementen zusammengesetzt sein, die von verschiedenen natürlich vorkommenden Promotoren abgeleitet sind oder können sogar aus synthetischen DNA-Segmenten zusammengesetzt sein. Ein Promotor kann auch DNA-Sequenzen enthalten, die an der Bindung von Proteinfaktoren beteiligt sind, die die Effizienz der Transkriptionsinitiation als Antwort auf physiologische oder entwicklungsbedingte Bedingungen kontrollieren.

Promotorelemente, insbesondere TATA-Elemente, die inaktiv sind oder eine stark verminderte Promotor-Aktivität in Abwesenheit einer stromaufwärtigen Aktivierung besitzen, werden als Minimalpromotoren oder Kernpromotoren bezeichnet. In Gegenwart eines geeigneten Transkriptionfaktors bzw. geeigneter Transkriptionsfaktoren liegt die Funktion des Minimalpromotors in der Ermöglichung der Transkription. Ein Minimal- oder Kernpromotor besteht somit nur aus allen Grundelementen, die für die Transkriptionsinitiation notwendig sind, z. B. einer TATA-Box und/oder einem Initiator.

Die Erfindung betrifft auch erfindungsgemäße DNA-Sequenzen enthaltende Vektorkonstrukte. Diese Vektorkonstrukte umfassen beliebige Plasmide, Cosmide, Phagen und andere Vektoren in doppelsträngiger oder einzelsträngiger, linearer oder zirkulärer Form, die gegebenenfalls selbst transmittierbar oder mobilisierbar sein können und die einen prokaryotischen oder eukaryotischen Wirt entweder durch Integration in das zelluläre Genom transformieren können oder die extrachromosomal vorliegen (z. B. autonom replizierende Plasmide mit einem Replikationsorigin).

Vektoren, Plasmide, Cosmide, künstliche Hefechromosomen (YACs), künstliche Bakterienchromosomen (BACs) und DNA-Segmente zur Verwendung zur Transformation von Zellen umfassen allgemein die die erfindungsgemäße Phospholipase codierende DNA sowie eine andere DNA, wie cDNA, ein Gen oder Gene, die in die Zellen eingeschleust werden sollen. Diese DNA-Konstrukte können weitere Strukturen wie Promotoren, Enhancer, Polylinker oder auch Regulatorgene, so weit erforderlich, umfassen. Eines der DNA-Segmente oder Gene, das bzw. die für die zelluläre Einschleusung ausgewählt wurde bzw. wurden, codiert/codieren zweckdienlicherweise ein Protein, das in den so erhaltenen transformierten (rekombinanten Zellen) exprimiert wird, was zu einem screenbaren oder selektierbaren Merkmal führt und/oder der transformierten Zelle einen verbesserten Phenotyp verleiht.

Die Konstruktion von Vektoren, die erfindungsgemäß verwendet werden können, ist einem Fachmann auf dem Gebiet angesichts der vorstehenden Offenbarung und des allgemeinen Fachwissens bekannt (vgl. z. B. Sambrook et al., Molecular Cloning: A Laboratory Manual (2. Aufl., Cold Spring Harbor Laboratory Press, Plainview, N.Y. (1989)).

Eine erfindungsgemäße Expressionskassette kann eine oder mehrere Restriktionsschnittstelle(n) enthalten, um das die Lysophospholipase codierende Polynucleotid unter die Regulation einer Regulatorsequenz zu stellen. Die Expressionskassette kann auch ein Terminationssignal in funktioneller Verknüpfung mit dem Polynucleotid sowie Regulatorsequenzen, die für die ordnungsgemäße Translation des Polynucleotids benötigt werden, enthalten. Die Expressionskassette, die das erfindungsgemäße Polynucleotid enthält, kann chimär sein, d.h. mindestens eine ihrer Komponenten ist bezogen auf mindestens eine der anderen Komponenten heterolog. Die Expression des Polynucleotids in der Expressionskassette kann unter der Kontrolle eines konstitutiven Promotors, eines induzierbaren Promotors, eines regulierten Promotors, eines viralen Promotors oder eines synthetischen Promotors stehen.

Die Vektoren können bereits Regulatorelemente enthalten, z.B. Promotoren, oder die erfindungsgemäßen DNA-Sequenzen können so manipuliert werden, dass sie solche Elemente enthalten. Geeignete Promotorelemente, die verwendet werden können, sind auf dem Fachgebiet bekannt und sind beispielsweise für *Trichoderma reesei* der cbh1- oder der cbh2-Promotor, für *Aspergillus oryzae* der amy-Promotor, für *Aspergillus niger* der xyl-, glaA-, alcA-, aphA-, tpiA-, gpdA-, sucl- und der pkiA-Promotor. Geeignete Promotorelemente, die zur Expression in Hefe verwendet werden können, sind auf dem Fachgebiet bekannt und sind beispielsweise der pho5-Promotor oder der gap-Promotor zur Expression in *Saccharomyces cerevisiae* und für *Pichia pastoris,* z.B. der aoxl-Promotor oder der fmd-Promotor oder der mox-Promotor für *H. polymorpha.*

DNA, die zur Einschleusung in Zellen geeignet ist, kann neben der erfindungsgemäßen DNA auch DNA umfassen, die aus einer beliebigen Quelle abgeleitet wurde oder daraus isoliert ist. Ein Beispiel für eine abgeleitete DNA ist eine DNA-Sequenz, die als nützliches Fragment in einem gegebenen Organismus identifiziert wurde und die dann in im Wesentlichen reiner Form chemisch synthetisiert wurde. Ein Beispiel für eine solche DNA ist eine geeignete DNA-Sequenz, die beispielsweise unter Verwendung von Restriktionsendonucleasen erhalten wurde, so dass sie weiter erfindungsgemäß manipuliert, beispielsweise amplifiziert werden kann. Zu diesen zählt unter anderem das als Markergen verwendbare amdS-Gen aus *Aspergillus nidulans* und seine regulatorische Sequenzen, sowie Polylinker.

Eine derartige DNA wird üblicherweise als rekombinante DNA bezeichnet. Daher umfasst eine geeignete DNA vollständig synthetische DNA, semisynthetische DNA, aus biologischen Quellen isolierte DNA und von eingeschleuster RNA abgeleitete DNA. Im Allgemeinen ist die eingeschleuste DNA kein ursprünglicher Bestandteil des Genotyps der Empfänger-DNA, aber erfindungsgemäß kann auch ein Gen aus einem gegebenen Genotyp isoliert und eventuell verändert werden und anschließend können Mehrfachkopien des Gens in den gleichen Genotyp eingeschleust werden, z. B. um die Produktion eines gegebenen Genprodukts zu verstärken.

Die eingeschleuste DNA umfasst ohne Beschränkung DNA aus Genen, wie beispielsweise aus Bakterien, Hefen, Pilzen oder Viren. Die eingeschleuste DNA kann modifizierte oder synthetische Gene, Teile von Genen oder chimäre Gene einschließlich Genen aus dem gleichen oder einem unterschiedlichen Genotyp umfassen. Hierzu kann z.B. auch DNA der Plasmide pUC18, pUC19 gehören.

Die zur Transformation erfindungsgemäß verwendete DNA kann zirkulär oder linear, doppelsträngig oder einzelsträngig sein. Im Allgemeinen liegt die DNA in Form einer chimären DNA wie eine Plasmid-DNA vor, die auch codierende Regionen enthält, die von Regulatorsequenzen flankiert sind und die Expression der in der transformierten Zelle vorhandenen rekombinanten DNA unterstützen. Beispielsweise kann die DNA selbst einen Promotor enthalten oder daraus bestehen, der in einer Zelle aktiv ist, der von einer Quelle, die sich von der Zelle unterscheidet, abgeleitet ist oder es kann ein Promotor verwendet werden, der bereits in der Zelle, d.h. der Transformationszielzelle, vorhanden ist.

Im Allgemeinen ist die eingeschleuste DNA relativ klein, weniger als etwa 30 kb, um die Empfindlichkeit gegenüber physikalischem, chemischem oder enzymatischem Abbau zu minimieren, der mit der Größe der DNA zunimmt.

Die Selektion eines geeigneten Expressionsvektors hängt von den Wirtszellen ab. Hefe- oder Pilzexpressionsvektoren können einen Replikationsorigin, einen geeigneten Promotor und Enhancer umfassen, und auch beliebige notwendige Ribosomenbindungsstellen, Polyadenylierungsstellen, Splice-Donor und -Akzeptor-Stellen, Transkriptionsterminationssequenzen und nicht-transkribierte 5'-flankierende Sequenzen umfassen.

Beispiele für geeignete Wirtszellen sind: Pilzzellen der Gattung *Aspergillus, Rhizopus, Trichoderma, Neurospora, Mucor, Penicillium,* etc., wie beispielsweise Hefen der Gattungen *Kluyveromyces, Saccharomyces, Schizosaccharomyces, Trichosporon, Schwanniomyces, Hansenula, Pichia* und dergleichen. Geeignete Wirtssysteme sind beispielsweise Pilze wie *Aspergilli,* z.B. *Aspergillus niger* (ATCC 9142) oder *Aspergillus ficuum* (NRLL 3135) oder *Trichoderma* (z.B. *Trichoderma reesei* QM6a) und Hefen wie *Saccharomyces,* z.B. *Saccharomyces cerevisiae* oder *Pichia,* wie z.B. *Pichia pastoris* oder *Hansenula,* z.B. *H. polymorpha* (DSMZ 70277). Derartige Mikroorganismen können von anerkannten Hinterlegungsstellen, z.B. der American Type Culture Collection (ATCC), dem Centraalbureau voor Schimmelcultures (CBS) oder der Deutschen Sammlung für Mikroorganismen und Zellkulturen GmbH (DSMZ) oder beliebigen anderen Hinterlegungsstellen erhalten werden.

Die Expressionskassette kann in der 5'-3'-Transkriptionsrichtung eine Transkriptions- und Translationstartregion des erfindungsgemäßen Polynucleotids und eine Transkriptions- und Terminationsregion, die in vivo oder in vitro funktionell ist, enthalten. Die Terminationsregion kann bezüglich der Transkriptionsinitiationsregion nativ sein oder kann bezüglich des Polynucleotids nativ oder anderer Herkunft sein. Die Regulatorsequenzen können stromaufwärts (5' nicht-codierende Sequenzen), innerhalb (Intron) oder stromabwärts (3' nicht-codierende Sequenzen) einer codierenden Sequenz lokalisiert sein und die Transkription, das RNA-Processing oder die Stabilität und/oder die Translation der assoziierten codierenden Sequenz beeinflussen. Regulatorsequenzen können ohne Beschränkung Enhancer, Promotoren, Repressorbindungsstellen, Translationsleadersequenzen, Introns oder Polyadenylierungsignalsequenzen umfassen. Sie können natürliche und synthetische Sequenzen sowie Sequenzen umfassen, die eine Kombination aus synthetischen und natürlichen Sequenzen sind.

Der erfindungsgemäß verwendete Vektor kann auch geeignete Sequenzen zur Amplifikation der Expression umfassen.

Beispiele für Promotoren, die erfindungsgemäß verwendet werden können, sind Promotoren, von denen bekannt ist, dass sie die Expression in den eukaryotischen Zellen kontrollieren. Beliebige Promotoren mit der Fähigkeit zur Expression in Fadenpilzen können verwendet werden. Beispiele sind ein Promotor, der stark durch Stärke oder Zellulose induziert wird, z. B. ein Promotor für Glucoamylase oder $\alpha$-Amylase aus der Gattung *Aspergillus* oder Cellulase (Cellobiohydrolase) aus der Gattung *Trichoderma,* ein Promotor für Enzyme im glykolytischen Stoffwechselweg, wie beispielsweise Phosphoglyceratkinase (PGK) und Glycerinaldehyd-3-phosphat-dehydrogenase (GPD), etc. Bevorzugt ist der Cellobiohydrolase-I-, der Cellobiohydrolase-II-, der Amylase-, der Glucoamylase-, der Xylanase- oder der Enolase-Promotor.

Zusätzlich zur Verwendung eines speziellen Promotors können andere Typen von Elementen die Expression von Transgenen beeinflussen. Insbesondere wurde gezeigt, dass Introns das Potenzial zur Verstärkung der Transgenexpression besitzen.

Die Expressionskassette kann noch weitere Elemente umfassen, beispielsweise solche, die durch endogene oder exogene Elemente wie Zinkfinger-Proteine, einschließlich natürlich vorkommender Zinkfinger-Proteine oder chimärer Zinkfinger-Proteine, reguliert werden können.

Die erfindungsgemäß verwendete Expressionskassette kann ferner Enhancer-Elemente oder stromaufwärtige Promotor-Elemente enthalten.

Vektoren zur erfindungsgemäßen Verwendung können so konstruiert werden, dass sie ein Enhancer-Element enthalten. Die erfindungsgemäßen Konstrukte umfassen somit das Gen von Interesse zusammen mit einer 3'-DNA-Sequenz, die als Signal wirkt, um die Transkription zu terminieren und die Polyadenylierung der so erhaltenen mRNA zu erlauben. Es können beliebige Signalsequenzen verwendet werden, die die Sekretion aus dem gewählten Wirtsorganismus ermöglichen. Eine bevorzugte Signalsequenz ist die Lysophospholipase-Signalsequenz aus *Aspergillus fumigatus* oder daraus abgeleitete Signalsequenzen für die Sekretion aus filamentösen Pilzen.

Es kann auch eine spezielle Leadersequenz verwendet werden, da die DNA-Sequenz zwischen der Transkriptionsstartstelle und dem Start der codierenden Sequenz, d.h. der nicht-translatierten Leadersequenz die Genexpression beeinflussen kann. Bevorzugte Leadersequenzen umfassen Sequenzen, die die optimale Expression des angehefteten Gens steuern, d.h. sie umfassen eine bevorzugte Consensus-Leader-Sequenz, die die mRNA-Stabilität erhöht oder erhält und eine ungeeignete Translationsinitiation verhindert. Die Wahl solcher Sequenzen ist einem Fachmann auf dem Gebiet gut bekannt.

Um die Möglichkeit, der Identifikation der Transformanten zu verbessern, kann ein selektierbares oder screenbares Markergen in die Expressionskassette aufgenommen werden. Derartige Markergene sind einem Fachmann auf dem Gebiet gut bekannt.

Die Expressionskassette oder ein Vektorkonstrukt, das die Expressionskassette enthält, wird in eine Wirtszelle eingeführt. Eine Vielzahl von Techniken sind verfügbar und einem Fachmann auf dem Gebiet zur Einschleusung von Konstrukten in eine Wirtszelle gut bekannt. Die Transformation mikrobieller Zellen kann unter Verwendung von Polyethylenglykol, Calciumchlorid, viraler Infektion, DEAE-Dextran, Phageninfektionen, Elektroporation und anderen auf dem Fachgebiet bekannten Methoden durchgeführt werden. Die Transformation von Pilzen kann nach Penttilä et al., Gene 61:155-164, 1987 durchgeführt werden. Die Einschleusung eines rekombinanten Vektors in Hefen kann nach an sich bekannten Verfahren einschließlich Elektroporation, Verwendung von Sphäroplasten, Lithiumacetat und dergleichen durchgeführt werden.

Sobald die erfindungsgemäße Expressionskassette bzw. DNA-Sequenz erhalten ist, kann sie in Vektoren nach an sich bekannten Verfahren eingefügt werden, um das codierte Polypeptid in geeigneten Wirtssystemen zu überexprimieren. Jedoch können auch DNA-Sequenzen als solche verwendet werden, um geeignete Wirtssysteme der Erfindung zu transformieren, um eine Überexpression des codierten Polypeptids zu erzielen.

Sobald eine erfindungsgemäße DNA-Sequenz in einer geeigneten Wirtszelle in einem geeigneten Medium exprimiert wurde, kann die codierte Lysophospholipase entweder aus dem Medium, falls die Lysophospholipase in das Medium sezerniert wird oder aus dem Wirtsorganismus, falls die Lysophospholipase intrazellulär z. B. im periplasmatischen Raum vorhanden ist, nach an sich bekannten Verfahren aufkonzentriert und/oder isoliert werden. Bekannte Verfahren der Abtrennung der unlöslichen Bestandteile des Kulturmediums und der Biomasse gefolgt von Verfahren zur Aufkonzentrierung der Lysophopholipase können zur Herstellung von konzentrierten Lysophospholipaselösungen oder als Vorbereitung zur Trocknung der Lysophospholipase angewendet werden. Beispielsweise können Filtrationsverfahren oder Zentrifugationsverfahren zur Abtrennung der unlöslichen Bestandteile verwendet werden gefolgt von Ultrafiltrationsverfahren zur Aufkonzentration oder es werden Cross-flow-Filtrationsverfahren angewendet. Die Trocknung kann durch Gefrier- und Sprühtrocknen, Granulationsverfahren, Extrudierung oder andere Verfahren erfolgen. Bekannte Verfahren der Proteinreinigung können verwendet werden, um die erfindungsgemäßen Lysophospholipasen zu isolieren. Beispielsweise können verschiedene chromatographische oder gelchromatographische Verfahren einzeln oder in Kombination verwendet werden. In Abhängigkeit von der verwendeten Wirtszelle bei einem rekombinanten Produktionsverfahren kann das erfindungsgemäße Enzym kovalent durch Glykosilierung modifiziert sein oder nicht. In eukaryotischen Zellen dient die Glykosilierung der sezernierten Proteine dazu, die Proteinfaltung, die Konformationsstabilität, die thermische Stabilität und die Resistenz gegenüber Proteolyse zu modulieren. Im Hinblick auf eine spezifische Anwendung der Lysophospholipase kann eine glykosilierte Variante des Enzyms gegenüber einer nicht-glykosilierten Variante bevorzugt sein.

Die Erfindung betrifft auch isolierte oder im Wesentlichen gereinigte Nucleinsäure- oder Proteinzusammensetzungen. Dabei bezeichnet ein isoliertes und gereinigtes Polynucleotid/Polypeptid bzw. Segment davon ein Polynucleotid bzw. Polypeptid bzw. Segment davon, das isoliert aus seiner nativen Umgebung und in gereinigter Form zur weiteren Verwendung vorliegt. Ein isoliertes Polynucleinsäuresegment oder Polypeptid kann in gereinigter Form vorliegen oder kann in einer nicht nativen Umgebung vorliegen, wie beispielsweise in einer transgenen Wirtszelle. Beispielsweise ist ein isoliertes oder gereinigtes Polynucleotidsegment oder Protein oder ein biologisch aktiver Teil davon im Wesentlichen frei von weiterem zellulärem Material oder Kulturmedium bei Produktion nach rekombinanten Techniken oder im Wesentlichen frei von chemischen Vorläufern oder anderen chemischen Verbindungen. Bevorzugt ist ein isoliertes Polynucleotid frei von Sequenzen (bevorzugt Protein-codierenden Sequenzen) die natürlicherweise die Nucleinsäure (d.h. Sequenzen, die an den 5'- und 3'-Enden der Nucleinsäure lokalisiert sind) in der genomischen DNA des Organismus aus dem die Nucleinsäure stammt, flankieren. Beispielsweise kann gemäß verschiedenen Ausführungsformen das isolierte Nucleinsäuremolekül weniger als etwa 5 kb, 4 kb, 3 kb, 2 kb, 1 kb, 0,5 kb oder 0,1 kb Nucleotidsequenzen enthalten, die natürlicherweise das Nucleinsäuremolekül in der genomischen DNA der Zelle, aus der die Nucleinsäure abgeleitet ist, flankieren. Ein Protein, das im Wesentlichen frei von zellulärem Material ist, umfasst Zusammensetzungen von Protein oder Polypeptid mit weniger als etwa 70%, 50%, 30%, 20%, 10%, 5% (bezogen auf das Trockengewicht) von verunreinigendem Protein. Wenn das erfindungsgemäße Protein oder ein biologisch aktives Fragment davon rekombinant produziert wird, umfasst bevorzugt das Kulturmedium weniger als etwa 70%, 50%, 30%, 20%, 10% oder 5% (bezogen auf das Trockengewicht) der chemischen Vorläufer oder nicht-proteinartigen chemischen Substanzen.

Die Erfindung betrifft auch Lysophospholipasezusammensetzungen, die das erfindungsgemäße Polypeptid enthalten. Im Allgemeinen sind Lysophospholipasezusammensetzungen flüssig oder trocken. Flüssige Zusammensetzungen enthalten das Lysophospholipaseenzym bevorzugt in einer gereinigten oder angereicherten Form. Jedoch können auch Hilfsstoffe wie beispielsweise ein Stabilisator und/oder Glycerin, Sorbit oder Monopropylenglykol, Additive wie Salze, Zucker, Konservierungsstoffe, Mittel zur Einstellung des pH-Werts und Proteine zugesetzt werden. Typische Flüssigzusammensetzungen sind wässrige oder ölige Aufschlämmungen.

Trockenzusammensetzungen können gefriergetrocknete, sprühgetrocknete, granulierte oder extrudierte Zusammensetzungen sein, die ausschließlich das Enzym enthalten können. Trockenzusammensetzungen können Granulate sein, die leicht mit anderen Komponenten gemischt werden können. Die Teilchengröße des Enzymgranulats ist bevorzugt mit der der anderen Komponenten des Gemisches kompatibel. Dies ermöglicht sichere und zweckdienliche Mittel, um Enzyme beispielsweise in zusammengesetzte Produkte einzuarbeiten.

Auf ähnliche Weise kann ein Nahrungsmitteladditiv gemäß dieser Ausführungsform der vorliegenden Erfindung mit anderen Nahrungsmittelkomponenten vereinigt werden, wodurch verarbeitete Nahrungsmittelprodukte erzeugt werden. Solche anderen Nahrungsmittelkomponenten umfassen ein oder mehrere Enzymsupplemente, Vitamine, Mineralien und Spurenelemente. Das so erhaltene kombinierte Nahrungsergänzungsmittel kann dann in einer geeigneten Menge mit anderen Nahrungsmittel-Komponenten wie Getreide und Pflanzenproteinen vermischt werden, um ein verarbeitetes Nahrungsmittel zu ergeben. Die Verarbeitung dieser Komponenten zu einem verarbeiteten Nahrungsmittel kann unter Verwendung an sich bekannter Verarbeitungsvorrichtungen durchgeführt werden.

In einer bevorzugten Ausführungsform umfassen die erfindungsgemäßen Lysophospholipasezusammensetzungen zusätzlich eine wirksame Menge eines oder mehrerer Enzyme für Nahrungs- oder Futtermittel oder für die Anwendung in Vorstufen zur Herstellung von Nahrungs- oder Futtermitteln, bevorzugt ausgewählt aus alpha-Galactosidasen, beta-Galactosidasen, Laccasen, anderen Phospholipasen, Phosphatasen, Endoglucanasen, insbesondere endo-beta-1,4-Glucanasen, endo-beta-1,3(4)-Glucanasen, endo-1,2-beta-Glucanasen und endo-1,3-alpha-Glucanasen, Cellulasen, Xylosidasen, Galactanasen, insbesondere A-rabinogalactan-endo-1,4-beta-Galactosidasen und Arabinogalactan-endo-1,3-beta-Galactosidasen, Pectin-abbauenden Enzymen, insbesondere Pectinasen, Pectinesterasen, Pectinlyasen, Polygalacturonasen, Arabananasen, Rhamnogalacturonasen, Rhamnogalacturonanacetylesterasen, Rhamnogalacturonan-alpha-Rhamnosidasen, Pectatlyasen und alpha-Galacturonidasen, Mannanasen, beta-Mannosidasen, Mannanacetylesterasen, Xylanacetylesterasen, Proteasen, Xylanasen, Arabinoxylanasen, lipolytischen Enzymen wie Lipasen, Digalactosid-Diglycerid Esterasen und Cutinasen, und andere Enzyme wie Laccasen und Transglutaminasen.

Die erfindungsgemäßen Lysophospholipasen können für eine Vielzahl von Anwendungen verwendet werden. Beispiele hierfür sind Anwendungen beim Verarbeitung von Weizenstärkehydrolysaten oder als Katalysatoren für die Umesterung bei Lysophospholipiden.

Die erfindungsgemäßen Lyophospholipasen können auch bei der Teigzubereitung von Brot- oder Kuchenteigen eingesetzt werden, um z.B. die Elastizität des Brotes oder Kuchens zu verbessern. Die Lysophospholipase kann dazu dem Teig während der Herstellung zugesetzt werden, gefolgt von den Schritten Teigkneten, Teigformen und Backen.

Die Lysophospholipase kann auch zusammen mit Phospholipasen zur Entschleimung von Öl eingesetzt werden, z.B. in einem Verfahren wie es z.B. in dem Patent EP 0 513 709 B2 (Röhm GmbH/Metallgesellschaft AG jetzt AB Enzymes GmbH/mg technologies ag) beschrieben ist.

Das Gen für die aus dem Mikroorganismus *Aspergillus fumigatus* isolierte isolierte Lysophospholipase wurde in dem Plasmid B6Sma35 unter der Hinterlegungsnummer 18370 am 14.06.2006 bei der Deutschen Sammlung von Mikroorganismen und Zellkulturen GmbH (DSMZ), Mascheroder Weg 1b, D-38124 Braunschweig gemäß den Bedingungen des Budapester Vertrags hinterlegt.

Die Erfindung wird anhand der beigefügten Figuren näher erläutert. Es zeigen:

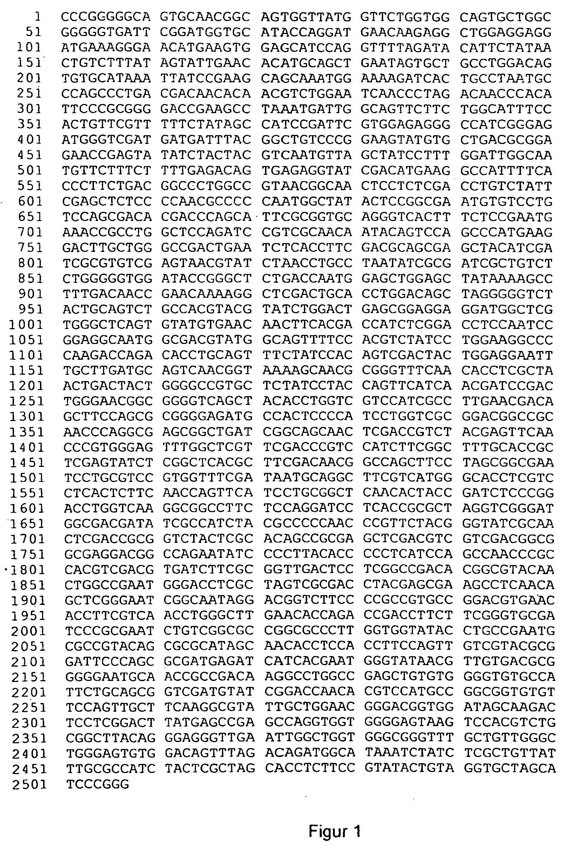

Figur 1: Die Nucleotidsequenz des chromosomalen Lysophospholipasegens aus dem Stamm *Aspergillus fumigatus* RH3949 (SEQ ID NO: 1)

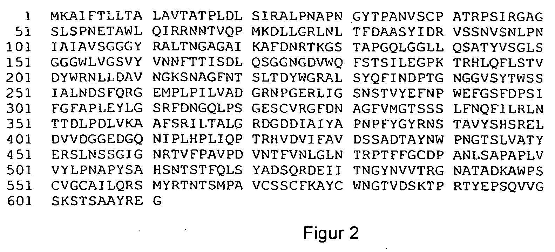

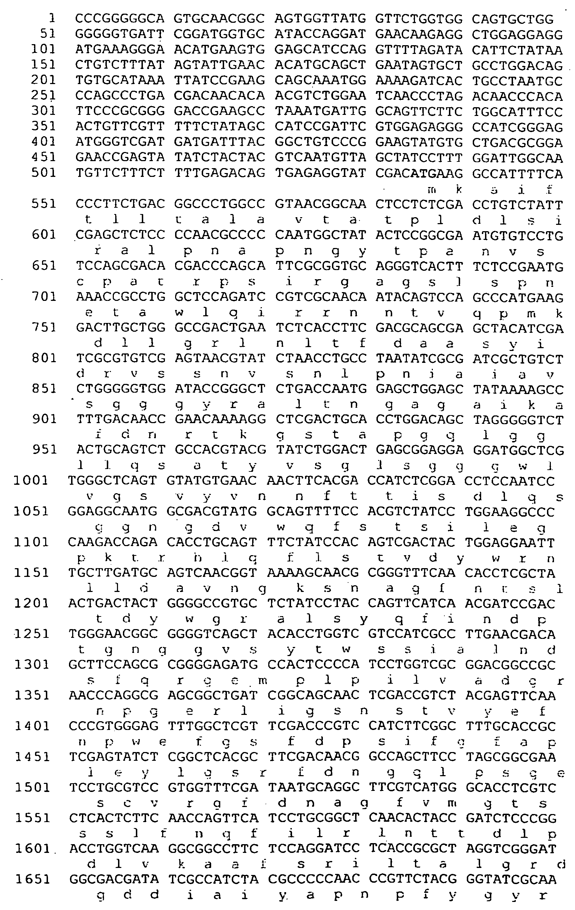

Figur 2: Die Aminosäuresequenz des Lysophospholipasegens aus dem Stamm *Aspergillus fumigatus* RH3949 (SEQ ID NO: 2)

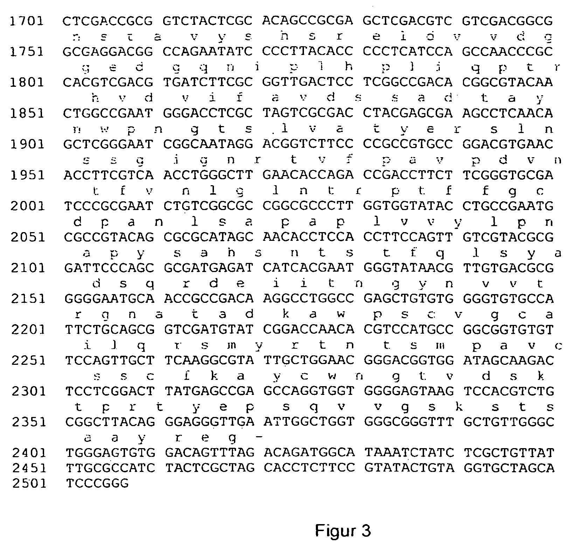

Figur 3: Die Nucleotidsequenz und daraus abgeleitete Aminosäuresequenz des chromosomalen Lysophospholipasegens aus dem Stamm *Aspergillus fumigatus* RH3949 (SEQ ID NOs: 1 und 2)

Figur 4: Die Restriktionskarte des Vektors B6Sma35

Figur 5: Die Restriktionskarte des Expressionsvektors pKB6Sma35d

Die folgenden Beispiele erläutern die Erfindung näher:

**Referenzbeispiel 1**

**Bestimmung der Lysophospholipaseaktivität**

1 Unit LPL entspricht der Enzymmenge, die in einer 0,01 molaren wässrigen Lösung von Lysolecithin bei pH 4,5 und 55°C eine Hydrolysegeschwindigkeit von 1 Mikromol pro Minute bewirkt.

Zur Bestimmung der Lysophospholipaseaktivität wird ein Gemisch aus 0,25 ml L-Lysophosphatidylcholin von Sigma Type 1 Cat.No. L4129 [10 mg L-Lysophosphatidylcholin in 1 ml dest .Wasser lösen (20 mmol)], 0,250 ml 0,02 M Natriumacetat Puffer pH 4,5 und 0,1 ml Enzymverdünnung in destilliertem Wasser bei 55°C für 10 min inkubiert. Puffer und Substratlösung werden für 5 min bei 55°C vorinkubiert. Proben werden nach 1 min und nach 10 min Reaktionszeit entnommen und die freigesetzten Fettsäuren mit Hilfe des optimierten enzymatischen Farbtests zur Bestimmung von freien Fettsäuren (Non-Esterified Fatty Acids, NEFA, Best.Nr 1383 175 von Fa. Boehringer-Mannheim) bestimmt. Als Reaktionszeit wird der Zeitraum zwischen der 1. und der 2. Probenentnahme benutzt.

**Referenzbeispiel 2**

**Bestimmung der Phospholipaseaktivität**

1 Phospholipase Unit entspricht der Enzymmenge, die unter Standardbedingungen 1 μmol Fettsäure pro Minute aus Phosphatidylcholin freisetzt.

**Reagenzien:**

**Substratlösung:**

1 g Epikuron 200 (gereinigtes Phosphatidylcholin aus Soja von LUCAS MEYER, BestNr. 139029), 100 ml deionisiertes-Wasser und 5 ml 0,32 M $CaCl_2$-Lösung werden mit einem *Ultra Turrax* 2 Min. bei 24000 UpM homogenisiert. Die Substratlösung ist bei 4°- 8°C 3 - 4 d haltbar.

**Andere Lösungen:**

0,32 M $MgCl_2$-Lösung, frische 3,3 mM Zitronensäure - Monohydrat- Lösung, 10 mM KOH-Lösung, 1%-ige Triton X100 (Fa. Fluka) Lösung in VE-Wasser

**Enzymlösung**

Die Enzympräparate werden in deionisiertem Wasser gelöst. Die Enzymkonzentration im Ansatz darf nicht über 2,5 U $g^{-1}$ enthalten.

**Durchführung der Bestimmung**

Hauptwerte

10 ml Substratlösung
10 ml 1%-ige Triton X100-Lösung
5 ml 3,3 mM Zitronensäure - Monohydrat- Lösung

werden in einen 25-ml-Weithalserlenmeyerkolben pipettiert und 10 min auf 40°C temperiert. Der pH-Wert stellt sich auf ca. 3,3 - 3,5 ein.

Nach der Zugabe von 0,1 ml Enzymlösung wird der Analysenansatz 10 min bei 40°C inkubiert. Nach Ablauf der Inkubationszeit wirld mit 0,01 M KOH auf pH 10,0 titriert, wobei die ersten 5 ml KOH zügig (Dauer: ca.1 min) zugegeben werden. Der Verbrauch an KOH wird registriert.

Blindwerte

Die Enzymstammlösung wird 15 min bei 95°C erhitzt und somit deaktiviert. Nach dem Abkühlen auf Raumtemperatur erfolgt die weitere Behandlung wie bei den Hauptwerten.

Eine Inkubation der Blindwerte ist nicht notwendig.

Auswertung:

$$PLU / g = \frac{\Delta V_{KOH} * c_{KOH} * 1000}{\Delta t * c_S * v}$$

| | | |
|---|---|---|
| $V_{KOH}$ | [ml] | Verbrauchsdifferenz zwischen Blind- und Hauptwert |
| $c_{KOH}$ | [mol $l^{-1}$] | Konzentration der KOH |
| t | [min ] | Inkubationszeit |
| $c_S$ | [g $ml^{-1}$] | Konzentration der Probe |
| v | [ml] | eingesetztes Volumen |

**Beispiel 1**

**Herstellung von Lysophospholipase mit *Aspergillus*** *fumigatus*

*Aspergillus fumigatus* RH3949 wurde in 200 ml Schüttelkolben, gefüllt mit 50 ml Medium bei 28°C, 200 UpM, über 5 d angezüchtet. Das Medium bestand aus 0,5% Epicuron 200 (Lucas Meyer), 0,5% Maisquellpulver, 0,2% $NH_4NO_3$, 100 mM $KH_2PO_4$ und 0,1% Triton X100. Der pH-Wert wurde vor dem Sterilisieren auf pH 6 eingestellt. Das Medium wurde mit einer Sporensuspension angeimpft. Nach 5 Tagen wurde der Kulturüberstand von dem Mycel durch Filtration getrennt und die Lysophospholipaseaktivität in der Flüssigkeit gemessen.

**Beispiel 2**

**Klonierung und Expression des Lysophospholipase (lpl) Gens aus dem *Aspergillus fumigatus* Stamm RH3949**

**a) DNA-Erststrangsynthese**

Ca. 1 x $10^7$ Sporen des *Aspergillus fumigatus* Stammes RH3949 wurden in 100 ml Medium (3,75% Glucidex, 3% Maisquellpulver, 0,5% $(NH_4)_2HPO_4$), beimpft und bei 45°C für 2 bis 5 Tage kultiviert. Das erhaltene Mycel wurde zur RNA-Präparation mittels der Qiagen-Säule (RNeasy Mini Plant Kit, Qiagen) verwendet.

Die cDNA-Erststrangsynthese erfolgte nach der Vorschrift des Herstellers (BRL). Hierbei wurden in einem 20 µl Reaktionsansatz 4 µl 5 x BRL Puffer (250 mM Tris/HCl, pH 8,3, 375 mM KCl, 15 mM $MgCl_2$) 1 µl 10 mM dNTP, 2 µl 100 mM DTT, 50 µMol Primer EA13, 1 µl RNA (2 µg gesamte RNA) und 2000 U RTase Super Script (BRL) zusammen pipettiert. Der Reaktionsansatz wurde für 50 min bei 45°C inkubiert.

Für die spätere Amplifikation der Lysophospholipase cDNA mittels der Polymerase-Ketten-Reaktion wurde der Ansatz mit 20 µl bidest Wasser verdünnt und bei -20°C aufbewahrt.

Die DNA-Sequenz des Primers EA13 lautet:

5'-gAC TCg AgT CgA CAT CgA $(T)_{20}$ (A/C/g)-3' (SEQ ID NO: 3)

**b) Amplifizierung einer Lysophospholipase cDNA-Teilsequenz mittels der Polymerase-Ketten-Reaktion (PCR)**

Aus dem Vergleich der Lysophospholipase-Aminosäuresequenzen von *S. cerevisiae* ( Lee et al., J. Biol. Chem., 1994, 269, 19725-19730), *P. notatum*-(Masuda et al., 1991, Eur. J. Biochem., 202, 783-787) und *Aspergillus awamori* RH3312 wurden verschiedene Oligoprimer für die Amplifizierung der Lysophospholipase-cDNA abgeleitet. Dabei zeigte sich, dass das Primerpaar 3949/875 und 3949/4710iv zu dem richtigen Lysophospholipase-cDNA Genfragment führt.

3949/875 5'-GAT GGC GGC GAG GAT GGA CAG AA-3' (SEQ ID NO: 4)
3949/8710iv 5'-AGT GCC GTT CCA GCA ATA-3'(SEQ ID NO: 5)

Aus dem Ansatz der cDNA-Erststrangsynthese wurde die Amplifizierung einer Lysophospholipase cDNA-Teilsequenz mittels der PCR Methode durchgeführt. Der Reaktionsansatz von 100 µl enthielt: 10 µl 10 x Puffer (200 mM Tris/HCl, pH 8,4, 500 mM KCl), 2 µl 10 mM dNTP, jeweils 50 pMol Oligoprimer, 1 µl des 1 Strang-cDNA Ansatzes, 5U Taq DNA-Polymerase (BRL). Der Ansatz wurde für die Denaturierung bei 95°C für 5 min, gefolgt von 45 Zyklen (95°C für 1 min, 45°C für 1 min, 72°C für 1 min) und anschließend die Extention bei 72°C für 5 min durchgeführt.

Die PCR-Produkte wurden über die Säule (Concert Rapid PCR Purification System, Gibco BRL) gereinigt und in pGEMT-Plasmid (Promega) kloniert.

Ein Transformant enthält die richtige Lysophospholipase cDNA-Teilsequenz und wurde als 3949/19/16 bezeichnet.

**c) Klonierung des chromosomalen Lysophospholipase (lpl) Gens aus dem Stamm RH3949**

Die chromosomale DNA Präparation wurde nach einer Vorschrift von Hynes, M.J et al. (1983) Mol. Cell. Biol. 3, 1430-1439, durchgeführt.

Nach partieller Hydrolyse mit Sau3A I wurde die DNA durch eine Saccharose-Dichtegradientenzentrifugation nach ihrer Größe fraktioniert. Fraktionen, die DNA-Fragmente von 9-20 kb enthielten, wurden zusammengefasst und mit Ethanol bei -20°C gefällt. Nach dem Waschen und Trocknen wurde die DNA in BamHI/EcoRI hydrolysierte EMBL3-DNA inseriert und in-vitro verpackt. Die Verpackung in das Phagenlysate Gigapack **III** Gold Packaging erfolgte nach der vom Hersteller beschriebenen Vorschrift (Stratagene Instruction Manual).

Zur Identifizierung des chromosomalen Lysophospholipase Gens in einer Lambda EMBL3-Genbank wurde das cDNA-Fragment aus dem Plasmid 3949/19/16 als radioaktive Gensonde verwendet. Die Hybridisierung wurde bei 65°C, 18 Stunden in Dextransulfat-Lösung (GenescreenPlus, DuPont) durchgeführt. Nach der Hybridisierung wurden die Filter jeweils 30 min zuerst mit 6 x SSC, zweimal 2x SSC, zweimal 2 x SSC, 0,1 % SDS und anschließend mit 0, 2 x SSC bei 65°C (Membrane transfer and detection methods, Amersham) gewaschen.

Sechs positive Klone wurden identifiziert. Aus den Ergebnissen der Analyse mit den Restriktionsendonucleasen und Southern-Hybridisierung wurde die Phagen-DNA des Klons B6 mit SmaI hydrolysiert. Das in pUC18 inserierte 2,5 kb SmaI-Fragment wurde als B6Sma35 bezeichnet (Figur 4). Die Nukleotidsequenz des 2,5 kb DNA-Fragments und die daraus abgeleitete Aminosäuresequenz wurden bestimmt (SEQ ID NOs: 1 und 2).

Das Gen enthält 2507 bp, ein Signalspeptid von 15 Aminosäuren: Met Lys Ala Ile Phe Thr Leu Leu Thr Ala Leu Ala Val Thr Ala (Prediction of Protein Localization Sites, PSORT, Nakai und Kanehisa, 1992, Genomics 14, 897-911) und hat ein berechnetes Molekulargewicht von 64068 Da. Ein Vergleich der Aminosäuresequenzen zwischen der erfindungsgemäßen *A. fumigatus* Lysophospholipase und der veröffentlichten PLB3 Lysophospholipase aus *A. fumigatus* zeigt eine Identität von 88% (BLAST-Bestimmung, http://www.ncbi.nlm.gov/blast. Altschul Stephen F., et. al.(1997), Gapped BLAST and PSI-Blast: a new generation of protein database search programs, Nucleic Acids Res. 25, 3389-3402 ).

**d) Konstruktion von Expressionsvektor pKB6Sma35d**

Zuerst wurde der N-terminale Bereich des Lysophospholipase Gens mittels der PCR Methode amplifiziert. Die dafür verwendeten Primer haben folgende Sequenz:

B6Sma3 5'-GAA TTC CGC GGA CTG CGC ATC ATG AAG GCC ATT TTC ACC CTT CTG AC-3' (SEQ ID NO: 6)

B6Sma4 5'-TGA GGA TCC TGG AGA AGG CCG CCT TG-3' (SEQ ID NO: 7)

Die Polymerase-Ketten-Reaktion erfolgte unter folgenden Bedingungen: Denaturierung bei 95°C für 5 min, 45 Zyklen bei 95°C für 1 min, 45°C für 1 min, 72°C für 1 min und Extention für 5 min bei 72°C. Der Reaktionsansatz von 100 $\mu$l enthielt: 10 $\mu$l 10 x PCR Puffer (200 mM Tris/HCl, pH 8,4, 500 mM KCl), 3 $\mu$l 50 mM $MgCl_2$, 2 $\mu$l 10 mM dNTP (je 10 mM dATP, dCTP, dGTP und dTTP), 50 pMol Oligoprimer, 10 ng Plasmid DNA als Matrize und 5 U Taq-DNA Polymerase (BRL). Das PCR Produkt wurde gereinigt, mit den Enzymen SacII / BamHI hydrolysiert und in das mit den selben Enzymen gespaltene Plasmid pALK487 inseriert. Durch die Sequenzierung des erhaltenen Plasmids pKB6Sma35b wurde die Richtigkeit der Klonierung bestätigt. Im gleichen Verfahren wurde der C-terminale Bereich des Gens mit dem Primerpaar B6Sma5 und Sma35/5 amplifiziert. Die Sequenz der Primer lautet:

Sma35/5 5'-ATG GGC ACC TCG TCC TCA CTC TTC-3' (SEQ ID NO: 8)
B6Sma5 5'-CGG GAT CCT AGC ACC TAC AG TAT ACG GAA G-3' (SEQ ID NO: 9)

Das PCR Produkt wurde nach der Hydrolyse mit BamHI in das mit dem gleichen Enzym gespaltene Plasmid pKB6Sma35b eingebaut. Das resultierende Plasmid mit der Bezeichnung pKB6Sma35c, welches das gesamte Lysophospholipase (lpl) Gen unter der Kontrolle des *T. reesei* cbhl-Promotors enthält, wurde präpariert und sequenziert.

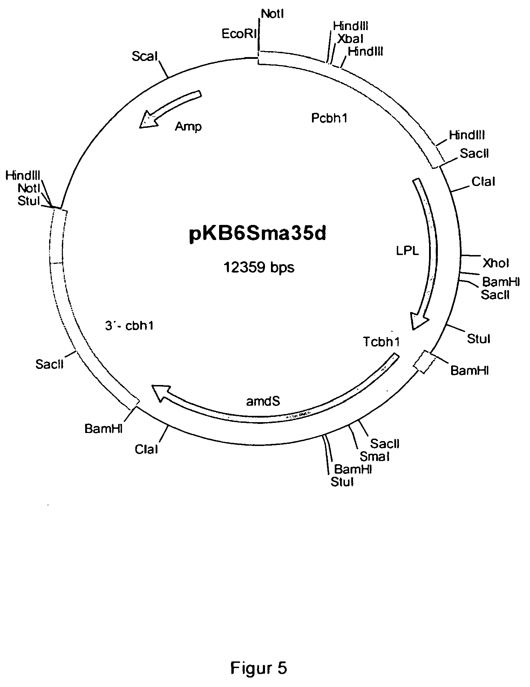

Durch Einbau des EcoRI, fill-in/ SpeI, fill-in Fragments aus dem Plasmid pALK424, welches den Selektionsmarker amdS enthält, in das mit dem Enzym SmaI gespaltene Plasmid pKB6Sma35c wurde der Expressionsvektor pKB6Sma35d konstruiert (Figur 5).

**e) Transformation von *T. reesei* Stamm RH31013**

Die zur Transformation verwendete DNA wurde aus dem Vektor pKB6Sma35d als NotI-Fragment isoliert. Dieses NotI-Fragment enthielt das Lysophospholipase-Gen unter Kontrolle des *T. reesei* cbhl-Promotors und den Selektionsmarker amdS.

Zur Isolierung des 9,3kb-Fragments wurde der Vektor pKB6Sma35d nach der Hydrolyse mit NotI in 1,2 % LowMelting Agarose elektrophoretisch getrennt. Das Agarose-Gel, welches das 9,7 kb NotI-DNA Fragment enthielt, wurde mit $\beta$-Agarase I (Biolabs) verdaut und danach die DNA mit Isopropanol gefällt. Für die Transformation werden zwischen 10 bis 15 $\mu$g DNA-Fragment benötigt.

Die Transformation in *T. reesei* RH 31013 wurde nach der Vorschrift von Penttilä M., et al. (1987, Gene 61, 155-64) durchgeführt.

Die nach der Transformation erhaltenen 13 Transformanten wurden zweimal über Einzelsporkolonien auf Selektionsplatten gereinigt und zuletzt auf PD-Medium (Kartoffel-Dextrose-Agar) übertragen. Die Transformanten RH31202 und RH31204 wurden für weitere Versuche benutzt.

**Beispiel 3**

**Rekombinante Herstellung von LPL aus *A. fumigatus* RH 3949**

Die Anzucht der Transformanten RH31202 und RH31204 erfolgte in 250 ml Schüttelkolben ohne Schikanen, mit 50 ml Medium (3% Lactose, 3% distillers spent grain, 5% $K_2HPO_4$, 0,5% $(NH_4)_2SO_4$ und 1% Maisquellpulver, pH-Wert 4,4). Das Medium wurde mit einer Sporensuspension inokuliert.

Nach 6 Tagen Inkubation bei 28°C, 200 UpM, 25 mm Auslenkung, wurde der Kulturüberstand von dem Mycel durch Sterilfiltration abgetrennt und für die weiteren Versuche benutzt.

**Beispiel 4**

**Charakterisierung der rekombinant hergestellten LPL aus *A. fumigatus***

Die Lysophospholipase- und die Phospholipaseaktivität der steril filtrierten Kulturlösungen von RH31202 und RH31204 wurden nach **Referenzbeispiel 1 und 2** bestimmt.

**Tab.: 1: Enzymaktivitäten der Kulturüberstände von RH 31202 und RH 31204 die die Lysophospholipase von *A. fumigatus* RH 3949 exprimieren.**

| Stamm | [LPL $g^{-1}$] | [PLU $g^{-1}$] | [LPL/PLU] |
|---|---|---|---|
| RH 31013 | 3 | 1 | 3 |
| RH 31202 | 22618 | 153 | 148 |
| RH 31204 | 22860 | 152 | 150 |

Die rekombinant produzierte Lysophospholipase aus *Aspergillus fumigatus* RH3949 hat ein sehr hohes LPL zu PL-Aktivitätsverhältnis. Das Enzym zeigt kaum Phospholipaseaktivität. Dies ist bei den in der Literatur beschriebenen Phospholipasen B nicht der Fall. Letztere zeigen ein geringeres Verhältnis LPL zu PL-Aktivität als das erfindungsgemäße Enzym.

**Beispiel 5**

**Verbesserung der Filtrierbarkeit von Maltosesirup**

Die rekombinant hergestellte LPL aus *Aspergillus fumigatus* RH3949 wurde in weiteren Versuchen zur Verbesserung der Filtration von Weizenstärkepartialhydrolysaten (kurz Sirup genannt) eingesetzt.

Teil A

Weizenstärkepartialhydrolysat der Firma Cerestar/Cargill (wheat B-starch) wurde bei 60° und 65°C für bis zu 4 h bei pH 4 mit Enzymen der Kulturüberstände aus dem Beispiel 3 inkubiert. Dazu wurde das Weizenstärkepartialhydrolysat mit HCl auf pH 4 eingestellt. 100 ml Weizenstärkepartialhydrolysat wurden in 200 ml Erlenmeyerkolben mit der LPL aus Beispiel 4 inkubiert bei einer Dosage von 280 LPL pro kg Sirup und die Lösung mit einem Magnetrührer gerührt. Während und am Ende der Inkubationsdauer wurde das Ergebnis visuell überprüft. Das Auftreten von Flocken, die eine klare Lösung bei Einwirkung von Lysophospholipase hinterlassen, zeigt die Wirkung des Enzyms an im Vergleich zum unbehandelten Sirup der trüb bleibt. Die Flockulierung wurde semiquantitativ in die Stufen sehr gut, gut, schlecht, keine Änderung eingestuft. Der auf 35°C abgekühlte Überstand wurde anschließend durch ein Papiefilter (Whatman No. 4) filtriert und die Filtrationsrate dabei nach 2,5 und 10 min bestimmt. Die Klarheit des Filtrates wurde photomoetrisch bei 720 nm bestimmt.

Bei Zusatz von erfindungsgemäßer Lysophospholipase wurde bereits nach 1 h eine gute Flockenbildung sichtbar, beim Blindwert ohne Enzymzusatz trat auch nach 4 h keine Flockenbildung auf und der Ansatz blieb trüb. Nach 4 h war bei dem Ansatz mit Enzym die Flockenbildung sehr gut.

Durch die Zugabe der erfindungsgemäßen Lysophospholipase wurde auch die Flitrationsgeschwindigkeit des enzymatisch behandelten Sirups deutlich erhöht wie aus Tabelle 2 ersichtlich ist.

**Tab.: 2: Behandlung von Weizenstärkepartialhydrolysat mit Lysophospholipase bei unterschiedlichen Temperaturen**

| Enzym | Temperatur [°C] | Dosage [LPL je kg Sirup] | Filtrat nach 2,5 min [ml] | Filtrat nach 10 min [ml] |
|---|---|---|---|---|
| Blindwert | 60°C | 0 | 7,5 | 11,5 |
| RH31202 | 60°C | 280 | 11,0 | 17,5 |
| | | | | |
| Blindwert | 65°C | 0 | 8,0 | 13,0 |
| RH31202 | 65°C | 280 | 14,0 | 23,0 |

Teil B

In einem weiteren Versuch wurde zu Weizenstärkepartialhydrolysat zusätzlich zu der erfindungsgemäßen Lysophospholipase auch β-Amylase zugesetzt wie dies in der Stärkeindustrie im großtechnischen Maßstab durchgeführt wird. Dabei wird der pH-Wert auf pH 4,9 mit HCl eingestellt und die Inkubationsdauer beträgt 6 h bei 60°C. Anschließend wurde die Flockulierung optisch beurteilt und die Filtrationsgeschwindigkeit wie im Teil A bestimmt. Die Ergebnisse des Filtrationstests sind in Tabelle 3 zusammengefasst.

**Tabelle 3: Behandlung von Weizstärkepartialhydrolysat mit ß-Amylase und Lysophospholipase bei pH 4,9, 60°C für 6 h. Die zugesetzte Menge an Lysophospholipase (RH 31202) betrug 280 LPL pro kg Sirup.**

| Enzym | Filtrat nach 2,5 min [ml] | Filtrat nach 10 min [ml] |
|---|---|---|
| Blindwert | 7,0 | 10,5 |
| Nur ß-Amylase | 14,5 | 25,0 |
| Lysophospholipase und ß-Amylase | 19,5 | 33,0 |

Die Ergebnisse zeigen, dass auch in Anwesenheit von β-Amylase, die zu einer weiteren Verringerung der Viskosität des Weizenstärkepartialhydrolysats führt, eine deutliche Steigerung der Filtrationsgeschwindigkeit durch den Zusatz der erfindungsgemäßen Lysophospholipase herbeigeführt wird.

Der Zusatz von ß-Amylase führt nicht zur Flockenbildung. Nur durch den Zusatz von Lysophospholipase kommt es zu einer sehr guten Flockenbildung.

Das Filtrat mit Zusatz von ß-Amylase hatte eine Extinktion von 0,116 AU bei 720 nm, wohingegen durch den Zusatz von Lysophospholipase die Extinktion des Filtrates auf 0,017 AU sank. Dies zeigt eine deutliche Verbesserung der Klarheit des Filtrats an, hervorgerufen durch den Zusatz von der erfindungsgemäßen Lysophospholipase.

## Patentansprüche

**1.** DNA-Sequenz, die ein Polypeptid mit Lysophospholipaseaktivität codiert, **dadurch gekennzeichnet, dass** die DNA-Sequenz ausgewählt ist aus

a) DNA-Sequenzen, die eine Nucleotidsequenz gemäß SEQ ID NO: 1 umfassen,
b) DNA-Sequenzen, die die codierende Sequenz gemäß SEQ ID NO: 1 umfassen,
c) DNA-Sequenzen, die die Proteinsequenz gemäß SEQ ID NO: 2 codieren,
d) DNA-Sequenzen, die von dem Plasmid B6Sma35 mit der Restriktionskarte gemäß Figur 4 und hinterlegt unter der Hinterlegungsnummer DSM 18370 codiert werden,
e) DNA-Sequenzen, die aufgrund der Degeneriertheit des genetischen Codes mit den DNA-Sequenzen gemäß a), b), c) oder d) verwandt sind, und
f) komplementären Strängen zu den Sequenzen gemäß a) bis e),

wobei die DNA-Sequenz bevorzugt aus *Aspergillus* und noch bevorzugter aus *Aspergillus fumigatus* abgeleitet ist und wobei das Polypeptid mit Lysophospholipaseaktivität bei einer Temperatur von 65°C, pH 5 in Weizenstärkepartialhydrolysat, über mindestens 4 h eine Aktivität von mindestens 80% beibehält.

2. Nucleinsäuresequenz, umfassend ein Analogon einer der Sequenzen gemäß Anspruch 1, **dadurch gekennzeichnet, dass** die Sequenz ein Polypeptid mit Lysophospholipaseaktivität codiert und

   a) mindestens 89% Identität zu einer dieser Sequenzen aufweist, oder
   b) mit einer dieser Sequenzen unter stringenten Bedingungen hybridisiert, oder
   c) ein komplementärer Strang zu den Sequenzen gemäß a) bis b) ist,

   wobei die Sequenz erhältlich ist durch Substitution, Deletion, Insertion, Addition oder Mutation einer oder mehrerer Nucleinsäuren der DNA-Sequenz gemäß SEQ ID NO: 1 und wobei das Polypeptid mit Lysophospholipaseaktivität bei einer Temperatur von 65°C, pH 5 in Weizenstärkepartialhydrolysat, über mindestens 4 h eine Aktivität von mindestens 80% beibehält.

3. Expressionskonstrukt umfassend eine Sequenz nach einem der Ansprüche 1 bis 2 in funktioneller Verknüpfung mit einer oder mehreren Sequenz(en) zur Steuerung der Expression des Polypeptids mit Lysophospholipaseaktivität in einer geeigneten Wirtszelle, wobei die Sequenz zur Steuerung der Expression des Polypeptids bevorzugt ein Promotor, ausgewählt aus dem Glucoamylase- oder $\alpha$-Amylase-Promotor der Gattung *Aspergillus,* dem Cellulase (Cellobiohydrolase-)-Promotor der Gattung *Trichoderma,* einem Promotor für ein Enzym im glykolytischen Stoffwechselweg wie Phosphoglyceratkinase oder Glycerinaldehyd-3-phosphatdehydrogenase, dem Xylanasepromotor oder dem Enolase-Promotor ist, und gegebenenfalls umfassend eine sekretorische Leadersequenz umfasst.

4. Rekombinante Wirtszelle, **dadurch gekennzeichnet, dass** sie mit einem Expressionskonstrukt nach Anspruch 3 transformiert worden ist.

5. Rekombinante Wirtszelle nach Anspruch 4, **dadurch gekennzeichnet, dass** sie von einer Pilzzelle der Gattung *Aspergillus, Rhizopus, Trichoderma, Neurospora, Mucor* oder *Penicillium* oder einer Hefezelle der Gattung *Kluyveromyces, Saccharomyces, Schizosaccharomyces, Trichosporon, Schwanniomyces, Hansenula* oder *Pichia* abgeleitet ist.

6. Plasmid B6Sma35 mit der Restriktionskarte gemäß Figur 4 und hinterlegt unter der Hinterlegungsnummer DSM 18370.

7. Polypeptid mit Lysophospholipaseaktivität ausgewählt aus

   a) einem Polypeptid, das von dem codierenden Teil einer DNA-Sequenz nach einem der Ansprüche 1 bis 2 codiert wird,
   b) einem Polypeptid mit einer Sequenz, die mindestens 89% Identität zu den Aminosäuren 16 bis 611 von SEQ ID NO: 2 aufweist, wobei das Polypeptid mit Lysophospholipaseaktivität bei einer Temperatur von 65°C, pH 5 in Weizenstärkepartialhydrolysat, über mindestens 4 h eine Aktivität von mindestens 80% beibehält.

8. Polypeptid mit Lysophospholipaseaktivität nach Anspruch 7, dadurch **ge - kennzeichnet,** dass es

   - ein Molekulargewicht im Bereich von 64 bis 78 kDa besitzt,
   - die Fettsäure aus Lysolecithin hydrolysieren kann,
   - ein hohes Verhältnis Lysophospholipaseaktivität zu Phospholipaseaktivität aufweist,
   - aus einem Organismus der Gattung *Aspergillus* isolierbar ist, und
   - immunologisch mit einem gegen die Sequenz SEQ ID NO: 2 gerichteten Antikörper reaktiv ist.

9. Polypeptid nach Anspruch 8, **dadurch gekennzeichnet, dass** es aus *Aspergillus fumigatus* isolierbar ist.

10. Polypeptid nach einem der Ansprüche 8 bis 9, dadurch **gekenn - zeichnet,** dass es die Sequenz gemäß SEQ ID NO: 2 umfasst.

11. Lysophospholipasezusammensetzung, **dadurch gekennzeichnet, dass** sie ein Polypeptid nach einem der Ansprüche 7 bis 10 zusammen mit Hilfsstoffen umfasst und dass sie gegebenenfalls weiterhin ein oder mehrere Enzyme für Nahrungs- oder Futtermittel umfasst.

12. Verwendung eines Polypeptids nach einem der Ansprüche 7 bis 10 oder einer Lysophospholipasezusammensetzung nach Anspruch 11 zur Verbesserung der Filtration von Sirup, bevorzugt von Sirup, ausgewählt aus Glucosesirup und Maltosesirup.

**13.** Verwendung eines Polypeptids nach einem der Ansprüche 7 bis 10 oder einer Lysophospholipasezusammensetzung nach Anspruch 11 zur Umesterung von Lysolecithinen.

**14.** Verfahren zur Produktion eines Polypeptids mit Lysophospholipaseaktivität nach einem der Ansprüche 7 bis 10, **dadurch gekennzeichnet, dass** man eine Wirtszelle nach einem der Ansprüche 4 oder 5 unter Bedingungen, die die Expression des Polypeptids fördern, züchtet und anschließend das Polypeptid gewinnt.

## Claims

**1.** A DNA sequence that encodes a polypeptide with lysophospholipase activity, **characterized in that** the DNA sequence is selected from

a) DNA sequences that comprise a nucleotide sequence according to SEQ ID NO: 1,
b) DNA sequences that comprise the coding sequence according to SEQ ID NO: 1,
c) DNA sequences that encode the protein sequence according to SEQ ID NO: 2,
d) DNA sequences that are encoded by the plasmid B6Sma35 with the restriction map according to Figure 4 and deposited under accession number DSM 18370,
e) DNA sequences that are related to the DNA sequences according to a), b), c) or d) due to the degeneracy of the genetic code, and
f) strands complementary to the sequences according to a) to e),

wherein the DNA sequence is preferably derived from *Aspergillus,* and more preferably from *Aspergillus fumigatus*; and wherein the polypeptide with lysophospholipase activity retains an activity of at least 80% at a temperature of 65°C, pH 5 in partial hydrolysate of wheat starch for at least 4 hrs.

**2.** A nucleic acid sequence that comprises an analogue of one of the sequences according to claim 1, **characterized in that** the sequence encodes a polypeptide with lysophospholipase activity, and

a) has at least 89% identity to one of these sequences, or
b) hybridizes with one of these sequences under stringent conditions or
c) is a strand complementary to the sequences according to a) to b)

wherein the sequence may be obtained by substitution, deletion, insertion, addition or mutation of one or more nucleic acid(s) of the DNA sequence according to SEQ ID NO: 1, and wherein the polypeptide with lysophospholipase activity retains an activity of at least 80% at a temperature of 65°C, pH 5 in partial hydrolysate of wheat starch for at least 4 hrs.

**3.** An expression construct that comprises a sequence according to any one of claims 1 to 2 in operable linkage with one or more sequence(s) to control the expression of the polypeptide with lysophospholipase activity in an appropriate host cell, wherein the sequence to control the expression of the polypeptide preferably is a promoter selected from the glucoamylase promoter or the a-amylase promoter of the genus *Aspergillus,* the cellulase (cellobiohydrolase) promoter of the genus *Trichoderma,* a promoter for an enzyme in the glycolytic metabolic pathway such as phosphoglycerate kinase or glycerol aldehyde-3-phosphate dehydrogenase, the xylanase promoter or the enolase promoter, and may comprise a secretory leader sequence.

**4.** A recombinant host cell, **characterized in that** it was transformed with an expression construct according to claim 3.

**5.** The recombinant host cell according to claim 4, **characterized in that** it is derived from a fungus cell of the genus *Aspergillus, Rhizopus, Trichoderma, Neurospora, Mucor* or *Penicillium* or a yeast cell of the genus *Kluyveromyces, Saccharomyces, Schizosaccharomyces, Trichosporon, Schwanniomyces, Hansenula* or *Pichia.*

**6.** The plasmid B6Sma35 with the restriction map according to Figure 4 and deposited under accession number DSM 18370.

**7.** A polypeptide with lysophospholipase activity selected from

a) a polypeptide that is encoded by the coding part of a DNA sequence according to any one of claims 1 to 2,
b) a polypeptide with a sequence, which has at least 89% identity with amino acids 16 to 611 of SEQ ID NO: 2, wherein the polypeptide with lysophospholipase activity retains an activity of at least 80% at a temperature of 65°C, pH 5 in partial hydrolysate of wheat starch for at least 4 hrs.

**8.** A polypeptide with lysophospholipase activity according to claim 7, **characterized in that** it

- has a molecular weight in the range of 64 to 78 kDa,
- may hydrolyze the fatty acid from lysolecithin,
- shows a high ratio of lysophospholipase activity to phospholipase activity
- may be isolated from an organism of the genus *Aspergillus.*
- is immunologically reactive with an antibody directed against the sequence SEQ ID NO: 2.

**9.** The polypeptide according to 8, **characterized in that** it may be isolated from *Aspergillus fumigatus.*

**10.** The polypeptide according to any one of claims 8 to 9, **characterized in that** it comprises the sequence according to SEQ ID NO: 2.

**11.** A lysophospholipase composition, **characterized in that** it comprises a polypeptide according to any one of claims 7 to 10 together with additives, and may further comprise one or more enzyme(s) for food or animal feed.

**12.** The use of a polypeptide according to any one of claims 7 to 10 or a lysophospholipase composition according to 11 for improving the filtration of syrup, preferably syrup selected from glucose syrup and maltose syrup.

**13.** The use of a polypeptide according to any one of claims 7 to 10 or a lysophospholipase composition according to claim 11 for the transesterification of lysolecithins.

**14.** The process for the production of a polypeptide with lysophospholipase activity according to any one of claims 7 to 10, **characterized in that** a host cell according to any one of claims 4 or 5 is grown under conditions that support the expression of the polypeptide and subsequently the polypeptide is recovered.

**Revendications**

**1.** Séquence d'ADN qui code pour un polypeptide présentant une activité de lysophospholipase, **caractérisée en ce que** la séquence d'ADN est choisie parmi :

a) des séquences d'ADN qui comprennent une séquence nucléotidique selon SEQ ID N° : 1,
b) des séquences d'ADN qui comprennent la séquence codante selon SEQ ID N° : 1,
c) des séquences d'ADN qui codent pour la séquence protéique selon SEQ ID N° : 2
d) des séquences d'ADN qui sont codées par le plasmide 86Sma35 présentant la carte de restriction selon la figure 4 et sont enregistrées sous le numéro d'enregistrement DSM 18370,
e) des séquences d'ADN qui, en raison de la dégénérescence du code génétique, sont apparentées aux séquences d'ADN selon a), b), c) ou d), et
f) des brins complémentaires aux séquences selon a) à e),

la séquence d'ADN étant dérivée de préférence *d'Aspergillus* et de manière encore davantage préférée, *d'Aspergillus fumigatus* et le polypeptide présentant une activité de lysophospholipase à une température de 65 °C, pH 5 dans un hydrolysat partiel d'amidon de blé conservant pendant au moins 4 h, une activité d'au moins 80 %.

**2.** Séquence d'acide nucléique comprenant un analogue de l'une des séquences selon la revendication 1, **caractérisée en ce que** la séquence code pour un polypeptide présentant une activité de lysophospholipase et

a) présente une identité d'au moins 89 % avec l'une de ces séquences, ou
b) s'hybride avec l'une de ces séquences dans des conditions stringentes, ou
c) est un brin complémentaire aux séquences selon a) à b), la séquence étant obtenue par substitution, délétion, insertion, addition ou mutation d'un ou plusieurs acides nucléiques de la séquence d'ADN selon SEQ ID N° : 1 et le polypeptide présentant une activité de lysophospholipase à une température de 65 °C, pH 5 dans un hydrolysat partiel d'amidon de blé conservant pendant au moins 4 h, une activité d'au moins 80 %.

**3.** Construction d'expression comprenant une séquence selon l'une des revendications 1 à 2 en liaison fonctionnelle avec une ou plusieurs séquence(s) pour commander l'expression du polypeptide ayant une activité de lysophospholipase dans une cellule hôte appropriée, la séquence pour la commande de l'expression du polypeptide étant de préférence un promoteur choisi parmi le promoteur de la glucoamylase ou de l'$\alpha$-amylase du genre *Aspergillus,* le promoteur de la cellulase (cellobiohydrolase) du genre *Trichoderma,* un promoteur pour une enzyme dans la voie métabolique glycolytique telle que la phosphoglycérate kinase ou la glycérinaldéhyde-3-phosphate-déshydrogénase, le promoteur de la xylanase ou le promoteur de l'énolase, et comprenant éventuellement une séquence leader sécrétoire.

**4.** Cellule hôte recombinante, **caractérisée en ce qu'**elle a été transformée avec une construction d'expression selon la revendication 3.

**5.** Cellule hôte recombinante selon la revendication 4, **caractérisée en ce qu'**elle est dérivée d'une cellule de champignon du genre *Aspergillus, Rhizopus, Trichoderma, Neurospora, Mucor ou Penicillium* ou une cellule de levure du genre *Kluyveromyces, Saccharomyces, Schizosaccharomyces, Trichosporon, Schwanniomyces, Hansenula* ou *Pichia.*

**6.** Plasmide B6Sma35 présentant la carte de restriction selon la figure 4 et enregistré sous le numéro d'enregistrement DSM 18370.

**7.** Polypeptide présentant une activité de lysophospholipase choisi parmi

a) un polypeptide qui est codé par la partie codante d'une séquence d'ADN selon l'une des revendications 1 à 2,
b) un polypeptide comportant une séquence qui présente une identité d'au moins 89 % avec les acides aminés 16 à 611 de SEQ ID N° : 2, le polypeptide ayant une activité de lysophospholipase à une température de 65 °C, pH 5 dans un hydrolysat partiel d'amidon de blé conservant pendant au moins 4 h, une activité d'au moins 80 %.

**8.** Polypeptide présentant une activité de lysophospholipase selon la revendication 7, **caractérisé en ce que**

- il possède un poids moléculaire dans la plage de 64 à 78 kDa,
- il peut hydrolyser l'acide gras de la lysolécithine,
- il présente un rapport élevé de l'activité de la lysophospholipase à l'activité de la phospholipase,
- il peut être isolé d'un organisme du genre *Aspergillus,* et
- il est réactif immunologiquement avec un anticorps dirigé contre la séquence SEQ ID N° : 2.

**9.** Polypeptide selon la revendication 8, **caractérisé en ce qu'**il peut être isolé *d'Aspergillus fumigatus.*

**10.** Polypeptide selon l'une des revendications 8 à 9, **caractérisé en ce qu'**il comprend la séquence selon SEQ ID N° : 2.

**11.** Composition de lysophospholipase, **caractérisée en ce qu'**elle comprend un polypeptide selon l'une des revendications 7 à 10 conjointement à des adjuvants, et **en ce qu'**elle comprend éventuellement en outre une ou plusieurs enzymes pour des aliments ou des aliments pour animaux.

**12.** Utilisation d'un polypeptide selon l'une des revendications 7 à 10 ou d'une composition de lysophospholipase selon la revendication 11, pour l'amélioration de la filtration d'un sirop, de préférence d'un sirop choisi parmi le sirop de glucose et le sirop de maltose.

**13.** Utilisation d'un polypeptide selon l'une des revendications 7 à 10 ou d'une composition de lysophospholipase selon la revendication 11, pour la transestérification des lysolécithines.

**14.** Procédé de production d'un polypeptide présentant une activité de lysophospholipase selon l'une des revendications 7 à 10, **caractérisé en ce que** l'on cultive une cellule hôte selon l'une des revendications 4 ou 5, dans des conditions qui stimulent l'expression du polypeptide, et ensuite l'on recueille le polypeptide.

| | | | | | |
|---|---|---|---|---|---|
| 1 | CCCGGGGGCA | GTGCAACGGC | AGTGGTTATG | GTTCTGGTGG | CAGTGCTGGC |
| 51 | GGGGGTGATT | CGGATGGTGC | ATACCAGGAT | GAACAAGAGG | CTGGAGGAGG |
| 101 | ATGAAAGGGA | ACATGAAGTG | GAGCATCCAG | GTTTTAGATA | CATTCTATAA |
| 151 | CTGTCTTTAT | AGTATTGAAC | ACATGCAGCT | GAATAGTGCT | GCCTGGACAG |
| 201 | TGTGCATAAA | TTATCCGAAG | CAGCAAATGG | AAAAGATCAC | TGCCTAATGC |
| 251 | CCAGCCCTGA | CGACAACACA | ACGTCTGGAA | TCAACCCTAG | ACAACCCACA |
| 301 | TTCCCGCGGG | GACCGAAGCC | TAAATGATTG | GCAGTTCTTC | TGGCATTTCC |
| 351 | ACTGTTCGTT | TTTCTATAGC | CATCCGATTC | GTGGAGAGGG | CCATCGGGAG |
| 401 | ATGGGTCGAT | GATGATTTAC | GGCTGTCCCG | GAAGTATGTG | CTGACGCGGA |
| 451 | GAACCGAGTA | TATCTACTAC | GTCAATGTTA | GCTATCCTTT | GGATTGGCAA |
| 501 | TGTTCTTTCT | TTTGAGACAG | TGAGAGGTAT | CGACATGAAG | GCCATTTTCA |
| 551 | CCCTTCTGAC | GGCCCTGGCC | GTAACGGCAA | CTCCTCTCGA | CCTGTCTATT |
| 601 | CGAGCTCTCC | CCAACGCCCC | CAATGGCTAT | ACTCCGGCGA | ATGTGTCCTG |
| 651 | TCCAGCGACA | CGACCCAGCA | TTCGCGGTGC | AGGGTCACTT | TCTCCGAATG |
| 701 | AAACCGCCTG | GCTCCAGATC | CGTCGCAACA | ATACAGTCCA | GCCCATGAAG |
| 751 | GACTTGCTGG | GCCGACTGAA | TCTCACCTTC | GACGCAGCGA | GCTACATCGA |
| 801 | TCGCGTGTCG | AGTAACGTAT | CTAACCTGCC | TAATATCGCG | ATCGCTGTCT |
| 851 | CTGGGGGTGG | ATACCGGGCT | CTGACCAATG | GAGCTGGAGC | TATAAAAGCC |
| 901 | TTTGACAACC | GAACAAAAGG | CTCGACTGCA | CCTGGACAGC | TAGGGGGTCT |
| 951 | ACTGCAGTCT | GCCACGTACG | TATCTGGACT | GAGCGGAGGA | GGATGGCTCG |
| 1001 | TGGGCTCAGT | GTATGTGAAC | AACTTCACGA | CCATCTCGGA | CCTCCAATCC |
| 1051 | GGAGGCAATG | GCGACGTATG | GCAGTTTTCC | ACGTCTATCC | TGGAAGGCCC |
| 1101 | CAAGACCAGA | CACCTGCAGT | TTCTATCCAC | AGTCGACTAC | TGGAGGAATT |
| 1151 | TGCTTGATGC | AGTCAACGGT | AAAAGCAACG | CGGGTTTCAA | CACCTCGCTA |
| 1201 | ACTGACTACT | GGGGCCGTGC | TCTATCCTAC | CAGTTCATCA | ACGATCCGAC |
| 1251 | TGGGAACGGC | GGGGTCAGCT | ACACCTGGTC | GTCCATCGCC | TTGAACGACA |
| 1301 | GCTTCCAGCG | CGGGGAGATG | CCACTCCCCA | TCCTGGTCGC | GGACGGCCGC |
| 1351 | AACCCAGGCG | AGCGGCTGAT | CGGCAGCAAC | TCGACCGTCT | ACGAGTTCAA |
| 1401 | CCCGTGGGAG | TTTGGCTCGT | TCGACCCGTC | CATCTTCGGC | TTTGCACCGC |
| 1451 | TCGAGTATCT | CGGCTCACGC | TTCGACAACG | GCCAGCTTCC | TAGCGGCGAA |
| 1501 | TCCTGCGTCC | GTGGTTTCGA | TAATGCAGGC | TTCGTCATGG | GCACCTCGTC |
| 1551 | CTCACTCTTC | AACCAGTTCA | TCCTGCGGCT | CAACACTACC | GATCTCCCGG |
| 1601 | ACCTGGTCAA | GGCGGCCTTC | TCCAGGATCC | TCACCGCGCT | AGGTCGGGAT |
| 1651 | GGCGACGATA | TCGCCATCTA | CGCCCCCAAC | CCGTTCTACG | GGTATCGCAA |
| 1701 | CTCGACCGCG | GTCTACTCGC | ACAGCCGCGA | GCTCGACGTC | GTCGACGGCG |
| 1751 | GCGAGGACGG | CCAGAATATC | CCCTTACACC | CCCTCATCCA | GCCAACCCGC |
| 1801 | CACGTCGACG | TGATCTTCGC | GGTTGACTCC | TCGGCCGACA | CGGCGTACAA |
| 1851 | CTGGCCGAAT | GGGACCTCGC | TAGTCGCGAC | CTACGAGCGA | AGCCTCAACA |
| 1901 | GCTCGGGAAT | CGGCAATAGG | ACGGTCTTCC | CCGCCGTGCC | GGACGTGAAC |
| 1951 | ACCTTCGTCA | ACCTGGGCTT | GAACACCAGA | CCGACCTTCT | TCGGGTGCGA |
| 2001 | TCCCGCGAAT | CTGTCGGCGC | CGGCGCCCTT | GGTGGTATAC | CTGCCGAATG |
| 2051 | CGCCGTACAG | CGCGCATAGC | AACACCTCCA | CCTTCCAGTT | GTCGTACGCG |
| 2101 | GATTCCCAGC | GCGATGAGAT | CATCACGAAT | GGGTATAACG | TTGTGACGCG |
| 2151 | GGGGAATGCA | ACCGCCGACA | AGGCCTGGCC | GAGCTGTGTG | GGGTGTGCCA |
| 2201 | TTCTGCAGCG | GTCGATGTAT | CGGACCAACA | CGTCCATGCC | GGCGGTGTGT |
| 2251 | TCCAGTTGCT | TCAAGGCGTA | TTGCTGGAAC | GGGACGGTGG | ATAGCAAGAC |
| 2301 | TCCTCGGACT | TATGAGCCGA | GCCAGGTGGT | GGGGAGTAAG | TCCACGTCTG |
| 2351 | CGGCTTACAG | GGAGGGTTGA | ATTGGCTGGT | GGGCGGGTTT | GCTGTTGGGC |
| 2401 | TGGGAGTGTG | GACAGTTTAG | ACAGATGGCA | TAAATCTATC | TCGCTGTTAT |
| 2451 | TTGCGCCATC | TACTCGCTAG | CACCTCTTCC | GTATACTGTA | GGTGCTAGCA |
| 2501 | TCCCGGG | | | | |

Figur 1

```
  1  MKAIFTLLTA LAVTATPLDL SIRALPNAPN GYTPANVSCP ATRPSIRGAG
 51  SLSPNETAWL QIRRNNTVQP MKDLLGRLNL TFDAASYIDR VSSNVSNLPN
101  IAIAVSGGGY RALTNGAGAI KAFDNRTKGS TAPGQLGGLL QSATYVSGLS
151  GGGWLVGSVY VNNFTTISDL QSGGNGDVWQ FSTSILEGPK TRHLQFLSTV
201  DYWRNLLDAV NGKSNAGFNT SLTDYWGRAL SYQFINDPTG NGGVSYTWSS
251  IALNDSFQRG EMPLPILVAD GRNPGERLIG SNSTVYEFNP WEFGSFDPSI
301  FGFAPLEYLG SRFDNGQLPS GESCVRGFDN AGFVMGTSSS LFNQFILRLN
351  TTDLPDLVKA AFSRILTALG RDGDDIAIYA PNPFYGYRNS TAVYSHSREL
401  DVVDGGEDGQ NIPLHPLIQP TRHVDVIFAV DSSADTAYNW PNGTSLVATY
451  ERSLNSSGIG NRTVFPAVPD VNTFVNLGLN TRPTFFGCDP ANLSAPAPLV
501  VYLPNAPYSA HSNTSTFQLS YADSQRDEII TNGYNVVTRG NATADKAWPS
551  CVGCAILQRS MYRTNTSMPA VCSSCFKAYC WNGTVDSKTP RTYEPSQVVG
601  SKSTSAAYRE G
```

Figur 2

```
   1  CCCGGGGGCA GTGCAACGGC AGTGGTTATG GTTCTGGTGG CAGTGCTGG
  51  GGGGGTGATT CGGATGGTGC ATACCAGGAT GAACAAGAGG CTGGAGGAGG
 101  ATGAAAGGGA ACATGAAGTG GAGCATCCAG GTTTTAGATA CATTCTATAA
 151  CTGTCTTTAT AGTATTGAAC ACATGCAGCT GAATAGTGCT GCCTGGACAG
 201  TGTGCATAAA TTATCCGAAG CAGCAAATGG AAAAGATCAC TGCCTAATGC
 251  CCAGCCCTGA CGACAACACA ACGTCTGGAA TCAACCCTAG ACAACCCACA
 301  TTCCCGCGGG GACCGAAGCC TAAATGATTG GCAGTTCTTC TGGCATTTCC
 351  ACTGTTCGTT TTTCTATAGC CATCCGATTC GTGGAGAGGG CCATCGGGAG
 401  ATGGGTCGAT GATGATTTAC GGCTGTCCCG GAAGTATGTG CTGACGCGGA
 451  GAACCGAGTA TATCTACTAC GTCAATGTTA GCTATCCTTT GGATTGGCAA
 501  TGTTCTTTCT TTTGAGACAG TGAGAGGTAT CGACATGAAG GCCATTTTCA
                                             m  k   a  i  f
 551  CCCTTCTGAC GGCCCTGGCC GTAACGGCAA CTCCTCTCGA CCTGTCTATT
        t  l   l  t  a  l   a  v  t   a  t  p   l  d  l  s  i
 601  CGAGCTCTCC CCAACGCCCC CAATGGCTAT ACTCCGGCGA ATGTGTCCTG
       r  a  l   p  n  a   p  n  g  y   t  p  a   n  v  s
 651  TCCAGCGACA CGACCCAGCA TTCGCGGTGC AGGGTCACTT TCTCCGAATG
      c  p  a  t   r  p  s   i  r  g   a  g  s  l   s  p  n
 701  AAACCGCCTG GCTCCAGATC CGTCGCAACA ATACAGTCCA GCCCATGAAG
       e  t  a   w  l  q  i   r  r  n   n  t  v   q  p  m  k
 751  GACTTGCTGG GCCGACTGAA TCTCACCTTC GACGCAGCGA GCTACATCGA
       d  l  l   g  r  l   n  l  t  f   d  a  a   s  y  i
 801  TCGCGTGTCG AGTAACGTAT CTAACCTGCC TAATATCGCG ATCGCTGTCT
      d  r  v  s   s  n  v   s  n  l   p  n  i  a   i  a  v
 851  CTGGGGGTGG ATACCGGGCT CTGACCAATG GAGCTGGAGC TATAAAAGCC
      s  g  g   g  y  r  a   l  t  n   g  a  g   a  i  k  a
 901  TTTGACAACC GAACAAAAGG CTCGACTGCA CCTGGACAGC TAGGGGGTCT
       f  d  n   r  t  k   g  s  t  a   p  g  q   l  g  g
 951  ACTGCAGTCT GCCACGTACG TATCTGGACT GAGCGGAGGA GGATGGCTCG
      l  l  q  s   a  t  y   v  s  g   l  s  g  g   g  w  l
1001  TGGGCTCAGT GTATGTGAAC AACTTCACGA CCATCTCGGA CCTCCAATCC
       v  g  s   v  y  v  n   n  f  t   t  i  s   d  l  q  s
1051  GGAGGCAATG GCGACGTATG GCAGTTTTCC ACGTCTATCC TGGAAGGCCC
       g  g  n   g  d  v   w  q  f  s   t  s  i   l  e  g
1101  CAAGACCAGA CACCTGCAGT TTCTATCCAC AGTCGACTAC TGGAGGAATT
       p  k  t  r   h  i  q   f  l  s   t  v  d  y   w  r  n
1151  TGCTTGATGC AGTCAACGGT AAAAGCAACG CGGGTTTCAA CACCTCGCTA
       l  l  d   a  v  n  g   k  s  n   a  g  f   n  r  s  l
1201  ACTGACTACT GGGGCCGTGC TCTATCCTAC CAGTTCATCA ACGATCCGAC
       t  d  y   w  g  r   a  l  s  y   q  f  i   n  d  p
1251  TGGGAACGGC GGGGTCAGCT ACACCTGGTC GTCCATCGCC TTGAACGACA
       t  g  n  g   g  v  s   y  t  w   s  s  i  a   l  n  d
1301  GCTTCCAGCG CGGGGAGATG CCACTCCCCA TCCTGGTCGC GGACGGCCGC
       s  f  q   r  g  e  m   p  l  p   i  l  v   a  d  g  r
1351  AACCCAGGCG AGCGGCTGAT CGGCAGCAAC TCGACCGTCT ACGAGTTCAA
       n  p  g   e  r  l   i  g  s  n   s  t  v   y  e  f
1401  CCCGTGGGAG TTTGGCTCGT TCGACCCGTC CATCTTCGGC TTTGCACCGC
       n  p  w  e   f  g  s   f  d  p   s  i  f  g   f  a  p
1451  TCGAGTATCT CGGCTCACGC TTCGACAACG GCCAGCTTCC TAGCGGCGAA
       i  e  y   l  g  s  r   f  d  n   g  q  l   p  s  g  e
1501  TCCTGCGTCC GTGGTTTCGA TAATGCAGGC TTCGTCATGG GCACCTCGTC
       s  c  v   r  g  f   d  n  a  g   f  v  m   g  t  s
1551  CTCACTCTTC AACCAGTTCA TCCTGCGGCT CAACACTACC GATCTCCCGG
       s  s  l  f   n  q  f   i  l  r   l  n  t  t   d  l  p
1601  ACCTGGTCAA GGCGGCCTTC TCCAGGATCC TCACCGCGCT AGGTCGGGAT
       d  l  v   k  a  a  f   s  r  i   l  t  a   l  g  r  d
1651  GGCGACGATA TCGCCATCTA CGCCCCCAAC CCGTTCTACG GGTATCGCAA
       g  d  d   i  a  i   y  a  p  n   p  f  y   g  y  r
```

```
1701  CTCGACCGCG GTCTACTCGC ACAGCCGCGA GCTCGACGTC GTCGACGGCG
        n  s  t  a  v  y  s  h  s  r  e  i  d  v  v  d  g
1751  GCGAGGACGG CCAGAATATC CCCTTACACC CCCTCATCCA GCCAACCCGC
        g  e  d  g  q  n  i  p  l  h  p  l  i  q  p  t  r
1801  CACGTCGACG TGATCTTCGC GGTTGACTCC TCGGCCGACA CGGCGTACAA
        h  v  d  v  i  f  a  v  d  s  s  a  d  t  a  y
1851  CTGGCCGAAT GGGACCTCGC TAGTCGCGAC CTACGAGCGA AGCCTCAACA
        n  w  p  n  g  t  s  l  v  a  t  y  e  r  s  l  n
1901  GCTCGGGAAT CGGCAATAGG ACGGTCTTCC CCGCCGTGCC GGACGTGAAC
        s  s  g  i  g  n  r  t  v  f  p  a  v  p  d  v  n
1951  ACCTTCGTCA ACCTGGGCTT GAACACCAGA CCGACCTTCT TCGGGTGCGA
        t  f  v  n  l  g  l  n  t  r  p  t  f  f  g  c
2001  TCCCGCGAAT CTGTCGGCGC CGGCGCCCTT GGTGGTATAC CTGCCGAATG
        d  p  a  n  l  s  a  p  a  p  l  v  v  y  l  p  n
2051  CGCCGTACAG CGCGCATAGC AACACCTCCA CCTTCCAGTT GTCGTACGCG
        a  p  y  s  a  h  s  n  t  s  t  f  q  l  s  y  a
2101  GATTCCCAGC GCGATGAGAT CATCACGAAT GGGTATAACG TTGTGACGCG
        d  s  q  r  d  e  i  i  t  n  g  y  n  v  v  t
2151  GGGGAATGCA ACCGCCGACA AGGCCTGGCC GAGCTGTGTG GGGTGTGCCA
        r  g  n  a  t  a  d  k  a  w  p  s  c  v  g  c  a
2201  TTCTGCAGCG GTCGATGTAT CGGACCAACA CGTCCATGCC GGCGGTGTGT
        i  l  q  r  s  m  y  r  t  n  t  s  m  p  a  v  c
2251  TCCAGTTGCT TCAAGGCGTA TTGCTGGAAC GGGACGGTGG ATAGCAAGAC
        s  s  c  f  k  a  y  c  w  n  g  t  v  d  s  k
2301  TCCTCGGACT TATGAGCCGA GCCAGGTGGT GGGGAGTAAG TCCACGTCTG
        t  p  r  t  y  e  p  s  q  v  v  g  s  k  s  t  s
2351  CGGCTTACAG GGAGGGTTGA ATTGGCTGGT GGGCGGGTTT GCTGTTGGGC
        a  a  y  r  e  g  -
2401  TGGGAGTGTG GACAGTTTAG ACAGATGGCA TAAATCTATC TCGCTGTTAT
2451  TTGCGCCATC TACTCGCTAG CACCTCTTCC GTATACTGTA GGTGCTAGCA
2501  TCCCGGG
```

Figur 3

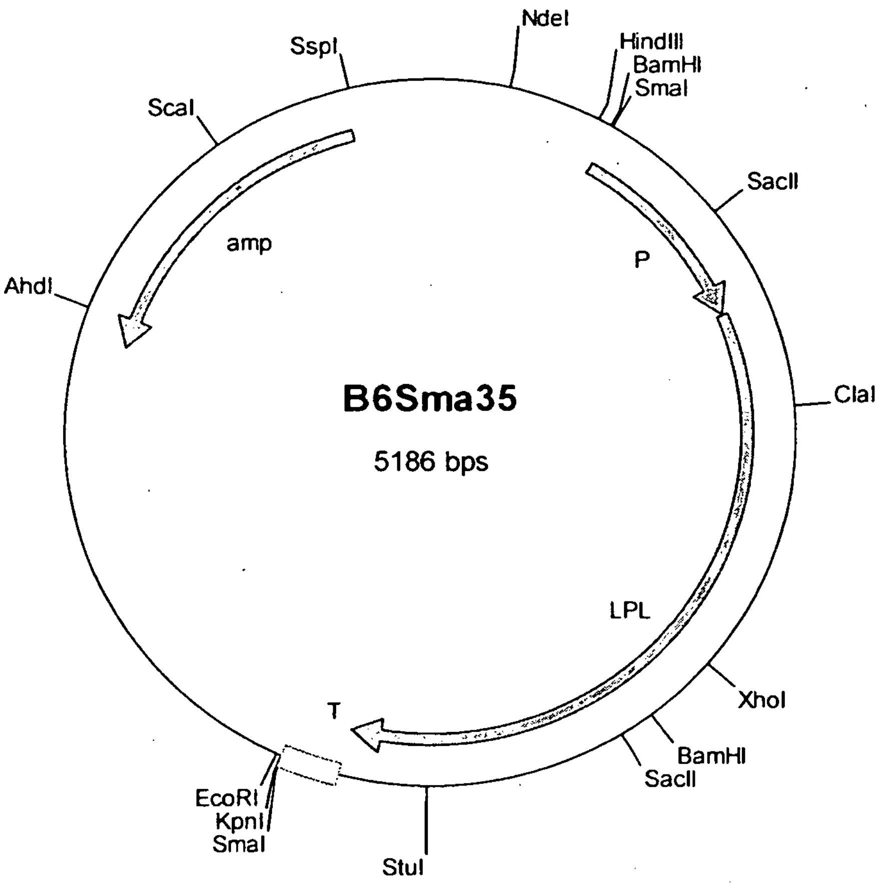
NdeI
HindIII
BamHI
SmaI
SspI
ScaI
SacII
amp
P
AhdI
B6Sma35
5186 bps
ClaI
LPL
XhoI
T
BamHI
SacII
EcoRI
KpnI
SmaI
StuI

Figur 4

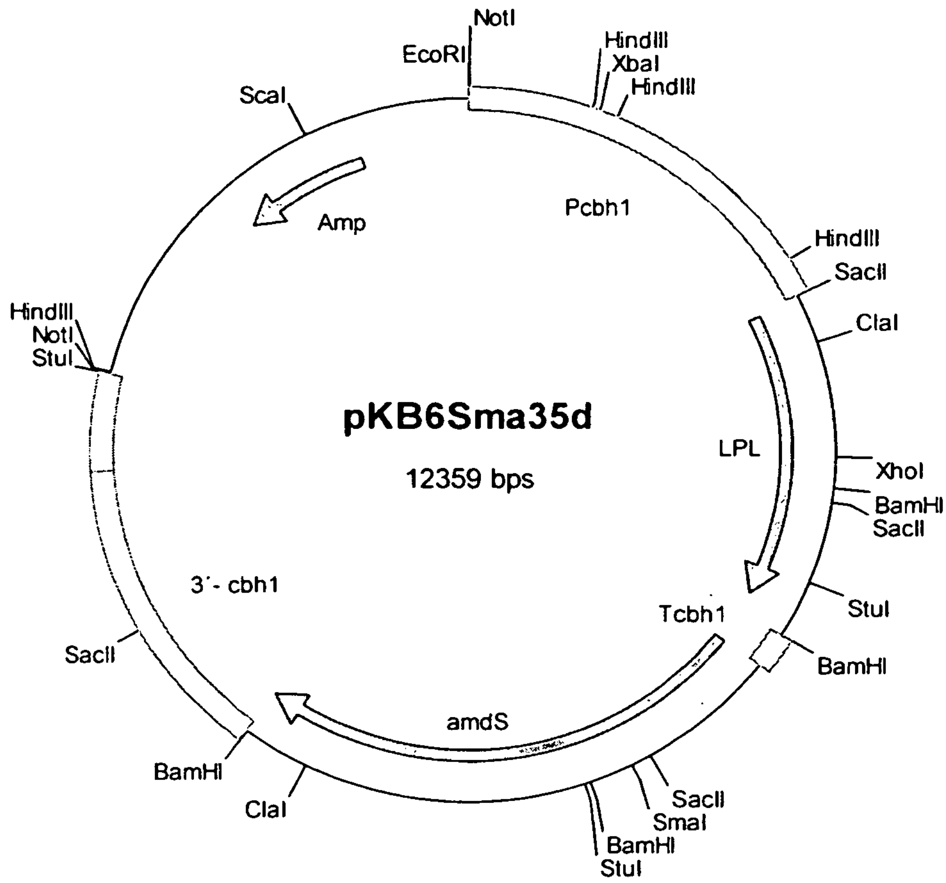
NotI
EcoRI
HindIII
XbaI
HindIII
ScaI
Amp
Pcbh1
HindIII
SacII
ClaI
HindIII
NotI
StuI
pKB6Sma35d
12359 bps
LPL
XhoI
BamHI
SacII
3´- cbh1
StuI
Tcbh1
BamHI
SacII
amdS
BamHI
ClaI
SacII
SmaI
BamHI
StuI

Figur 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0127251 A
- WO 03097825 A
- WO 0129222 A
- WO 0028044 A
- EP 0808903 A
- JP 03151879 B
- WO 9831790 AB
- WO 9826057 A
- JP 10155493 A
- WO 0224881 A
- EP 0575133 A2
- US 5538874 A
- US 5378623 A
- US 5521080 A
- WO 9818912 A
- WO 2004097012 A
- WO 0032758 A
- WO 03060112 A
- WO 2004111216 A
- WO 02066622 A
- EP 0219269 B1
- EP 99973065 A
- WO 01027251 A
- US 4873192 A
- EP 0513709 B2


### In der Beschreibung aufgeführte Nicht-Patentliteratur


- *FEMS Microbiol. Let.,* 1983, vol. 18, 15-18
- *Annu. Rev. Biochem.,* 1972, vol. 41, 129-160
- *Biochemistry,* 04. Mai 1999, vol. 38 (18), 5864-5871
- **SHEN et al.** *FEMS Microbiol Lett.,* 2004, vol. 239 (1), 87-93
- **N. MASUDA et al.** *Eur. J. Biochem.,* 1991, vol. 202, 783-787
- **LEE et al.** *J. Biol. Chem.,* 1994, vol. 269, 19725-19730
- **MERKEL et al.** *J. Biol. Chem.,* 1999, vol. 274, 28121-28127
- **WATANABE et al.** *FEMS Microbiology Letters,* 1994, vol. 124, 29-34
- **OISHI et al.** *Biosci. Biotechnol. Biochem.,* 1999, vol. 63, 83-90
- *J. Biol. Chem.,* 1998, vol. 273 (40), 26078-26086
- *Medical Mycology,* 1998, vol. 37, 61-67
- **WRIGHT LESLEY et al.** Cryptococcal phospholipases: a novel lysophospholipase discovered in the pathogenic fungus Cryptococcus gattii. *BIOCHEMICAL JOURNAL,* 01. Dezember 2004, vol. 384, 377-384
- **FUJINO SHUJI et al.** Purification and characterization of phospholipase B from Candida utilis. *BIOSCIENCE BIOTECHNOLOGY AND BIOCHEMISTRY,* Februar 2006, vol. 70 (2), 377-386
- **GHANNOUM MAHMOUD A.** Potential role of phospholipases in virulence and fungal pathogenesis. *CLINICAL MICROBIOLOGY REVIEWS,* Januar 2000, vol. 13 (1), 122-143
- **LEIDICH STEVEN D et al.** Cloning and disruption of caPLB1, a phospholipase B gene involved in the pathogenicity of Candida albicans. *JOURNAL OF BIOLO-GICAL CHEMISTRY,* 02. Oktober 1998, vol. 273 (40), 26078-26086
- **HOOVER CHARLES I et al.** Cloning and regulated expression of the Candida albicans phospholipase B (PLB1) gene. *FEMS MICROBIOLOGY LETTERS,* 15. Oktober 1998, vol. 167 (2), 163-169
- **SHEN et al.** *FEMS Microbiol. Letters,* 2004, vol. 239 (1), 87-93
- **HIRSCHBERG, D.S.** A linear space algorithm for computing longest common subsequences. *Commun Assoc Comput Mach,* 1975, vol. 18, 341-343
- **MYERS, E.W. ; W. MILLER.** Optimal alignments in linear space. *CABIOS,* 1988, vol. 4 (1), 11-17
- **CHAO, K-M ; W.R. PEARSON ; W. MILLER.** Aligning two sequences within a specified diagonal band. *CABIOS,* 1992, vol. 8 (5), 481-487
- **KUNKEL.** *Proc. Natl. Acad. Sci. USA,* 1985, vol. 82, 488
- **KUNKEL et al.** *Methods in Enzymol.,* 1987, vol. 154, 367
- Techniques in Molecular Biology. Mac Millan Publishing Company, 1983
- **VON DAYHOFF et al.** Atlas of Protein Sequence and Structure. Natl. Biomed. Res. Found, 1978
- **SAMBROOK et al.** Molecular Cloning: A Laboratory Manual. Cold Spring Harbor Laboratory Press, 1989
- **PENTTILÄ et al.** *Gene,* 1987, vol. 61, 155-164

- **MASUDA et al.** *Eur. J. Biochem.,* 1991, vol. 202, 783-787
- **VON HYNES, M.J et al.** *Mol. Cell. Biol.,* 1983, vol. 3, 1430-1439
- **NAKAI ; KANEHISA.** Prediction of Protein Localization Sites, PSORT. *Genomics,* 1992, vol. 14, 897-911
- **ALTSCHUL STEPHEN F.** Gapped BLAST and PSI-Blast: a new generation of protein database search programs. *Nucleic Acids Res.,* 1997, vol. 25, 3389-3402, http://www.ncbi.nlm.gov/blast
- **VON PENTTILÄ M. et al.** *Gene,* 1987, vol. 61, 155-64

SEQUENZPROTOKOLL

<110> AB ENZYMES GMBH

<120> KLONIERUNG, EXPRESSION UND VERWENDUNG SAURER LYPOPHOSPHOLIPASEN

<130> 16826WO

<160> 9

<170> PatentIn version 3.1

<210> 1
<211> 2507
<212> DNA
<213> Aspergillus fumigatus

<220>
<221> CDS
<222> (535)..(2370)
<223>

<400> 3

```
cccgggggca gtgcaacggc agtggttatg gttctggtgg cagtgctggc gggggtgatt      60
cggatggtgc ataccaggat gaacaagagg ctggaggagg atgaaaggga acatgaagtg     120
gagcatccag gttttagata cattctataa ctgtctttat agtattgaac acatgcagct     180
gaatagtgct gcctggacag tgtgcataaa ttatccgaag cagcaaatgg aaaagatcac     240
tgcctaatgc ccagccctga cgacaacaca acgtctggaa tcaaccctag acaacccaca     300
ttcccgcggg gaccgaagcc taaatgattg gcagttcttc tggcatttcc actgttcgtt     360
tttctatagc catccgattc gtggagaggg ccatcgggag atgggtcgat gatgatttac     420
ggctgtcccg gaagtatgtg ctgacgcgga gaaccgagta tatctactac gtcaatgtta     480
gctatccttt ggattggcaa tgttctttct tttgagacag tgagaggtat cgac atg       537
                                                            Met
                                                            1
```

```
aag gcc att ttc acc ctt ctg acg gcc ctg gcc gta acg gca act cct      585
Lys Ala Ile Phe Thr Leu Leu Thr Ala Leu Ala Val Thr Ala Thr Pro
            5                   10                  15

ctc gac ctg tct att cga gct ctc ccc aac gcc ccc aat ggc tat act      633
Leu Asp Leu Ser Ile Arg Ala Leu Pro Asn Ala Pro Asn Gly Tyr Thr
        20                  25                  30

ccg gcg aat gtg tcc tgt cca gcg aca cga ccc agc att cgc ggt gca      681
Pro Ala Asn Val Ser Cys Pro Ala Thr Arg Pro Ser Ile Arg Gly Ala
    35                  40                  45

ggg tca ctt tct ccg aat gaa acc gcc tgg ctc cag atc cgt cgc aac      729
Gly Ser Leu Ser Pro Asn Glu Thr Ala Trp Leu Gln Ile Arg Arg Asn
50                  55                  60                  65

aat aca gtc cag ccc atg aag gac ttg ctg ggc cga ctg aat ctc acc      777
Asn Thr Val Gln Pro Met Lys Asp Leu Leu Gly Arg Leu Asn Leu Thr
                70                  75                  80

ttc gac gca gcg agc tac atc gat cgc gtg tcg agt aac gta tct aac      825
Phe Asp Ala Ala Ser Tyr Ile Asp Arg Val Ser Ser Asn Val Ser Asn
            85                  90                  95

ctg cct aat atc gcg atc gct gtc tct ggg ggt gga tac cgg gct ctg      873
Leu Pro Asn Ile Ala Ile Ala Val Ser Gly Gly Gly Tyr Arg Ala Leu
        100                 105                 110

acc aat gga gct gga gct ata aaa gcc ttt gac aac cga aca aaa ggc      921
Thr Asn Gly Ala Gly Ala Ile Lys Ala Phe Asp Asn Arg Thr Lys Gly
    115                 120                 125

tcg act gca cct gga cag cta ggg ggt cta ctg cag tct gcc acg tac      969
Ser Thr Ala Pro Gly Gln Leu Gly Gly Leu Leu Gln Ser Ala Thr Tyr
130                 135                 140                 145

gta tct gga ctg agc gga gga gga tgg ctc gtg ggc tca gtg tat gtg     1017
Val Ser Gly Leu Ser Gly Gly Gly Trp Leu Val Gly Ser Val Tyr Val
                150                 155                 160

aac aac ttc acg acc atc tcg gac ctc caa tcc gga ggc aat ggc gac     1065
Asn Asn Phe Thr Thr Ile Ser Asp Leu Gln Ser Gly Gly Asn Gly Asp
            165                 170                 175

gta tgg cag ttt tcc acg tct atc ctg gaa ggc ccc aag acc aga cac     1113
Val Trp Gln Phe Ser Thr Ser Ile Leu Glu Gly Pro Lys Thr Arg His
        180                 185                 190

ctg cag ttt cta tcc aca gtc gac tac tgg agg aat ttg ctt gat gca     1161
Leu Gln Phe Leu Ser Thr Val Asp Tyr Trp Arg Asn Leu Leu Asp Ala
    195                 200                 205

gtc aac ggt aaa agc aac gcg ggt ttc aac acc tcg cta act gac tac     1209
Val Asn Gly Lys Ser Asn Ala Gly Phe Asn Thr Ser Leu Thr Asp Tyr
210                 215                 220                 225

tgg ggc cgt gct cta tcc tac cag ttc atc aac gat ccg act ggg aac     1257
Trp Gly Arg Ala Leu Ser Tyr Gln Phe Ile Asn Asp Pro Thr Gly Asn
                230                 235                 240
```

```
ggc ggg gtc agc tac acc tgg tcg tcc atc gcc ttg aac gac agc ttc      1305
Gly Gly Val Ser Tyr Thr Trp Ser Ser Ile Ala Leu Asn Asp Ser Phe
            245                 250                 255

cag cgc ggg gag atg cca ctc ccc atc ctg gtc gcg gac ggc cgc aac      1353
Gln Arg Gly Glu Met Pro Leu Pro Ile Leu Val Ala Asp Gly Arg Asn
        260                 265                 270

cca ggc gag cgg ctg atc ggc agc aac tcg acc gtc tac gag ttc aac      1401
Pro Gly Glu Arg Leu Ile Gly Ser Asn Ser Thr Val Tyr Glu Phe Asn
    275                 280                 285

ccg tgg gag ttt ggc tcg ttc gac ccg tcc atc ttc ggc ttt gca ccg      1449
Pro Trp Glu Phe Gly Ser Phe Asp Pro Ser Ile Phe Gly Phe Ala Pro
290                 295                 300                 305

ctc gag tat ctc ggc tca cgc ttc gac aac ggc cag ctt cct agc ggc      1497
Leu Glu Tyr Leu Gly Ser Arg Phe Asp Asn Gly Gln Leu Pro Ser Gly
                310                 315                 320

gaa tcc tgc gtc cgt ggt ttc gat aat gca ggc ttc gtc atg ggc acc      1545
Glu Ser Cys Val Arg Gly Phe Asp Asn Ala Gly Phe Val Met Gly Thr
            325                 330                 335

tcg tcc tca ctc ttc aac cag ttc atc ctg cgg ctc aac act acc gat      1593
Ser Ser Ser Leu Phe Asn Gln Phe Ile Leu Arg Leu Asn Thr Thr Asp
        340                 345                 350

ctc ccg gac ctg gtc aag gcg gcc ttc tcc agg atc ctc acc gcg cta      1641
Leu Pro Asp Leu Val Lys Ala Ala Phe Ser Arg Ile Leu Thr Ala Leu
    355                 360                 365

ggt cgg gat ggc gac gat atc gcc atc tac gcc ccc aac ccg ttc tac      1689
Gly Arg Asp Gly Asp Asp Ile Ala Ile Tyr Ala Pro Asn Pro Phe Tyr
370                 375                 380                 385

ggg tat cgc aac tcg acc gcg gtc tac tcg cac agc cgc gag ctc gac      1737
Gly Tyr Arg Asn Ser Thr Ala Val Tyr Ser His Ser Arg Glu Leu Asp
                390                 395                 400

gtc gtc gac ggc ggc gag gac ggc cag aat atc ccc tta cac ccc ctc      1785
Val Val Asp Gly Gly Glu Asp Gly Gln Asn Ile Pro Leu His Pro Leu
            405                 410                 415

atc cag cca acc cgc cac gtc gac gtg atc ttc gcg gtt gac tcc tcg      1833
Ile Gln Pro Thr Arg His Val Asp Val Ile Phe Ala Val Asp Ser Ser
        420                 425                 430

gcc gac acg gcg tac aac tgg ccg aat ggg acc tcg cta gtc gcg acc      1881
Ala Asp Thr Ala Tyr Asn Trp Pro Asn Gly Thr Ser Leu Val Ala Thr
    435                 440                 445

tac gag cga agc ctc aac agc tcg gga atc ggc aat agg acg gtc ttc      1929
Tyr Glu Arg Ser Leu Asn Ser Ser Gly Ile Gly Asn Arg Thr Val Phe
450                 455                 460                 465

ccc gcc gtg ccg gac gtg aac acc ttc gtc aac ctg ggc ttg aac acc      1977
Pro Ala Val Pro Asp Val Asn Thr Phe Val Asn Leu Gly Leu Asn Thr
                470                 475                 480
```

```
aga ccg acc ttc ttc ggg tgc gat ccc gcg aat ctg tcg gcg ccg gcg      2025
Arg Pro Thr Phe Phe Gly Cys Asp Pro Ala Asn Leu Ser Ala Pro Ala
            485                 490                 495

ccc ttg gtg gta tac ctg ccg aat gcg ccg tac agc gcg cat agc aac      2073
Pro Leu Val Val Tyr Leu Pro Asn Ala Pro Tyr Ser Ala His Ser Asn
        500                 505                 510

acc tcc acc ttc cag ttg tcg tac gcg gat tcc cag cgc gat gag atc      2121
Thr Ser Thr Phe Gln Leu Ser Tyr Ala Asp Ser Gln Arg Asp Glu Ile
    515                 520                 525

atc acg aat ggg tat aac gtt gtg acg cgg ggg aat gca acc gcc gac      2169
Ile Thr Asn Gly Tyr Asn Val Val Thr Arg Gly Asn Ala Thr Ala Asp
530                 535                 540                 545

aag gcc tgg ccg agc tgt gtg ggg tgt gcc att ctg cag cgg tcg atg      2217
Lys Ala Trp Pro Ser Cys Val Gly Cys Ala Ile Leu Gln Arg Ser Met
                550                 555                 560

tat cgg acc aac acg tcc atg ccg gcg gtg tgt tcc agt tgc ttc aag      2265
Tyr Arg Thr Asn Thr Ser Met Pro Ala Val Cys Ser Ser Cys Phe Lys
            565                 570                 575

gcg tat tgc tgg aac ggg acg gtg gat agc aag act cct cgg act tat      2313
Ala Tyr Cys Trp Asn Gly Thr Val Asp Ser Lys Thr Pro Arg Thr Tyr
        580                 585                 590

gag ccg agc cag gtg gtg ggg agt aag tcc acg tct gcg gct tac agg      2361
Glu Pro Ser Gln Val Val Gly Ser Lys Ser Thr Ser Ala Ala Tyr Arg
    595                 600                 605

gag ggt tga attggctggt gggcgggttt gctgttgggc tgggagtgtg              2410
Glu Gly
610

gacagtttag acagatggca taaatctatc tcgctgttat ttgcgccatc tactcgctag   2470

cacctcttcc gtatactgta ggtgctagca tcccggg                              2507
```

<210> 2
<211> 611
<212> PRT
<213> Aspergillus *fumigatus*

<400> 4

```
Met Lys Ala Ile Phe Thr Leu Leu Thr Ala Leu Ala Val Thr Ala Thr
1               5                   10                  15

Pro Leu Asp Leu Ser Ile Arg Ala Leu Pro Asn Ala Pro Asn Gly Tyr
            20                  25                  30
```

```
Thr Pro Ala Asn Val Ser Cys Pro Ala Thr Arg Pro Ser Ile Arg Gly
        35                  40                  45

Ala Gly Ser Leu Ser Pro Asn Glu Thr Ala Trp Leu Gln Ile Arg Arg
    50                  55                  60

Asn Asn Thr Val Gln Pro Met Lys Asp Leu Leu Gly Arg Leu Asn Leu
65                  70                  75                  80

Thr Phe Asp Ala Ala Ser Tyr Ile Asp Arg Val Ser Ser Asn Val Ser
                85                  90                  95

Asn Leu Pro Asn Ile Ala Ile Ala Val Ser Gly Gly Gly Tyr Arg Ala
            100                 105                 110

Leu Thr Asn Gly Ala Gly Ala Ile Lys Ala Phe Asp Asn Arg Thr Lys
        115                 120                 125

Gly Ser Thr Ala Pro Gly Gln Leu Gly Gly Leu Leu Gln Ser Ala Thr
    130                 135                 140

Tyr Val Ser Gly Leu Ser Gly Gly Gly Trp Leu Val Gly Ser Val Tyr
145                 150                 155                 160

Val Asn Asn Phe Thr Thr Ile Ser Asp Leu Gln Ser Gly Gly Asn Gly
                165                 170                 175

Asp Val Trp Gln Phe Ser Thr Ser Ile Leu Glu Gly Pro Lys Thr Arg
            180                 185                 190

His Leu Gln Phe Leu Ser Thr Val Asp Tyr Trp Arg Asn Leu Leu Asp
        195                 200                 205

Ala Val Asn Gly Lys Ser Asn Ala Gly Phe Asn Thr Ser Leu Thr Asp
    210                 215                 220

Tyr Trp Gly Arg Ala Leu Ser Tyr Gln Phe Ile Asn Asp Pro Thr Gly
225                 230                 235                 240

Asn Gly Gly Val Ser Tyr Thr Trp Ser Ser Ile Ala Leu Asn Asp Ser
                245                 250                 255

Phe Gln Arg Gly Glu Met Pro Leu Pro Ile Leu Val Ala Asp Gly Arg
            260                 265                 270

Asn Pro Gly Glu Arg Leu Ile Gly Ser Asn Ser Thr Val Tyr Glu Phe
        275                 280                 285
```

```
Asn Pro Trp Glu Phe Gly Ser Phe Asp Pro Ser Ile Phe Gly Phe Ala
    290                 295                 300

Pro Leu Glu Tyr Leu Gly Ser Arg Phe Asp Asn Gly Gln Leu Pro Ser
305                 310                 315                 320

Gly Glu Ser Cys Val Arg Gly Phe Asp Asn Ala Gly Phe Val Met Gly
                325                 330                 335

Thr Ser Ser Ser Leu Phe Asn Gln Phe Ile Leu Arg Leu Asn Thr Thr
            340                 345                 350

Asp Leu Pro Asp Leu Val Lys Ala Ala Phe Ser Arg Ile Leu Thr Ala
        355                 360                 365

Leu Gly Arg Asp Gly Asp Asp Ile Ala Ile Tyr Ala Pro Asn Pro Phe
    370                 375                 380

Tyr Gly Tyr Arg Asn Ser Thr Ala Val Tyr Ser His Ser Arg Glu Leu
385                 390                 395                 400

Asp Val Val Asp Gly Gly Glu Asp Gly Gln Asn Ile Pro Leu His Pro
                405                 410                 415

Leu Ile Gln Pro Thr Arg His Val Asp Val Ile Phe Ala Val Asp Ser
            420                 425                 430

Ser Ala Asp Thr Ala Tyr Asn Trp Pro Asn Gly Thr Ser Leu Val Ala
        435                 440                 445

Thr Tyr Glu Arg Ser Leu Asn Ser Ser Gly Ile Gly Asn Arg Thr Val
    450                 455                 460

Phe Pro Ala Val Pro Asp Val Asn Thr Phe Val Asn Leu Gly Leu Asn
465                 470                 475                 480

Thr Arg Pro Thr Phe Phe Gly Cys Asp Pro Ala Asn Leu Ser Ala Pro
                485                 490                 495

Ala Pro Leu Val Val Tyr Leu Pro Asn Ala Pro Tyr Ser Ala His Ser
            500                 505                 510

Asn Thr Ser Thr Phe Gln Leu Ser Tyr Ala Asp Ser Gln Arg Asp Glu
        515                 520                 525
```

```
Ile Ile Thr Asn Gly Tyr Asn Val Val Thr Arg Gly Asn Ala Thr Ala
    530                 535                     540

Asp Lys Ala Trp Pro Ser Cys Val Gly Cys Ala Ile Leu Gln Arg Ser
545                 550                 555                 560

Met Tyr Arg Thr Asn Thr Ser Met Pro Ala Val Cys Ser Ser Cys Phe
                565                 570                 575

Lys Ala Tyr Cys Trp Asn Gly Thr Val Asp Ser Lys Thr Pro Arg Thr
            580                 585                 590

Tyr Glu Pro Ser Gln Val Val Gly Ser Lys Ser Thr Ser Ala Ala Tyr
        595                 600                 605

Arg Glu Gly
    610
```

<210> 3
<211> 39
<212> DNA
<213> Artificial Sequence

<220>
<223> Primer

<400> 3
gactcgagtc gacatcgatt tttttttttt tttttttv 39

<210> 4
<211> 23
<212> DNA
<213> Artificial Sequence

<220>
<223> Primer

<400> 4
gatggcggcg aggatggaca gaa 23

<210> 5
<211> 18
<212> DNA
<213> Artificial Sequence

<220>
<223> Primer

<400> 5
agtgccgttc cagcaata 18

<210> 6
<211> 47
<212> DNA
<213> Artificial Sequence

<220>
<223> Primer

<400> 6
gaattccgcg gactgcgcat catgaaggcc attttcaccc ttctgac 47

<210> 7
<211> 26
<212> DNA
<213> Artificial Sequence

<220>
<223> Primer

<400> 7
tgaggatcct ggagaaggcc gccttg 26

<210> 8
<211> 24
<212> DNA
<213> Artificial Sequence

<220>
<223> Primer

<400> 8
atgggcacct cgtcctcact cttc 24

<210> 9
<211> 30
<212> DNA
<213> Artificial Sequence

<220>
<223> Primer

<400> 9
cgggatccta gcacctacag tatacggaag 30